(19) Europäisches Patentamt
European Patent Office
Office européen des brevets

(11) EP 3 138 137 B1

(12) # EUROPÄISCHE PATENTSCHRIFT

(45) Veröffentlichungstag und Bekanntmachung des Hinweises auf die Patenterteilung:
**20.06.2018 Patentblatt 2018/25**

(21) Anmeldenummer: **16736423.1**

(22) Anmeldetag: **04.07.2016**

(51) Int Cl.:
***H01L 51/54*** *(2006.01)* ***C07D 209/82*** *(2006.01)*
***C07D 417/10*** *(2006.01)* *C09K 11/06 (2006.01)*
*C07D 487/04 (2006.01)*

(86) Internationale Anmeldenummer:
**PCT/EP2016/065724**

(87) Internationale Veröffentlichungsnummer:
**WO 2017/005699 (12.01.2017 Gazette 2017/02)**

(54) **ORGANISCHE MOLEKÜLE ZUR VERWENDUNG IN OPTOELEKTRONISCHEN VORRICHTUNGEN**

ORGANIC MOLECULES FOR USE IN ORGANIC OPTOELECTRONIC DEVICES

MOLECULES ORGANIQUES DESTINEES A ETRE UTILISEES DANS DES DISPOSITIFS ORGANIQUES OPTOELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.07.2015 EP 15175358**
**17.12.2015 EP 15200813**
**09.05.2016 EP 16168821**

(43) Veröffentlichungstag der Anmeldung:
**08.03.2017 Patentblatt 2017/10**

(73) Patentinhaber: **CYNORA GMBH**
**76646 Bruchsal (DE)**

(72) Erfinder:
- **DANZ, Michael**
**76344 Eggenstein-Leopoldshafen (DE)**
- **ZINK, Daniel**
**76646 Bruchsal (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**US-A1- 2014 131 686**

- **SHAOLONG GONG ET AL: "Simple CBP isomers with high triplet energies for highly efficient blue electrophosphorescence", JOURNAL OF MATERIALS CHEMISTRY, ROYAL SOCIETY OF CHEMISTRY, GB, Bd. 22, Nr. 7, 1. Januar 2012 (2012-01-01) , Seiten 2894-2899, XP002734092, ISSN: 0959-9428, DOI: 10.1039/C1JM14903B [gefunden am 2011-12-21]**
- **NIKHIL REDDY MADADI ET AL: "Synthesis and anti-proliferative activity of aromatic substituted 5-((1-benzyl-1H-indol-3-yl)methylene)-1,3-dimethylpyrimidine-2,4,6(1H,3H,5H)-trione analogs against human tumor cell lines", BIOORGANIC & MEDICINAL CHEMISTRY LETTERS, Bd. 24, Nr. 2, 9. Dezember 2013 (2013-12-09), Seiten 601-603, XP55304756, AMSTERDAM, NL ISSN: 0960-894X, DOI: 10.1016/j.bmcl.2013.12.013**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 138 137 B1

## Beschreibung

Die Erfindung betrifft rein organische Moleküle und deren Verwendung in organischen lichtemittierenden Dioden (OLEDs) und in anderen organischen optoelektronischen Vorrichtungen.

## Hintergrund

Organische optoelektronische Vorrichtungen zeichnen sich dadurch aus, dass entweder elektrische Energie in Photonen umgewandelt werden (Organische Lichtemittierende Dioden, OLED oder Lichtemittierende Elektrochemische Zellen, LEEC) oder der umgekehrte Prozess abläuft (Organische Photovoltaik, OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Begrenzte Effizienzen von OLED-Materialien können durch Verwendung effizienter Materialien, die thermisch aktivierte verzögerte Fluoreszenz (TADF) zeigen, verbessert werden, da im Gegensatz zu rein fluoreszenten Materialien statt 25 % der in einer OLED gebildeten Exzitonen bis zu 100 % der Exzitonen genutzt werden können. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett- ($S_1$) und Triplett-Niveau ($T_1$). Dies kann beispielsweise durch Verwendung von Kupfer-(I)-Komplexen (siehe hierzu z. B.: H. Yersin, U. Monkowius, T. Fischer, T. Hofbeck, WO 2010/149748 A1) aber auch durch rein organische Materialien (siehe hierzu z. B.: Q. Zhang et al., J. Am. Chem. Soc. 2012, 134, 14706, WO 2013161437 A1) erreicht werden.

Es gibt weiterhin einen großen Bedarf an neuen Materialien, zum Beispiel nach tiefblauen TADF-OLEDs. Bestehende blaue TADF-Materialien zeigen oft hohe Exzitonenlebensdauern, welche schlecht für effiziente und langlebige OLEDs sind. Neben den erwähnten Eigenschaften der Materialien ist für eine Kommerzialisierung ebenso die Zugänglichkeit relevant. Dies schließt die Verfügbarkeit von Synthesebausteinen, als auch den Aufwand für die eigentliche Synthese des funktionellen Materials, insbesondere dessen Aufreinigung mit ein.

Aus der US 2014/0131686 A1 ist ein lichtemittierendes Element bekannt, welches eine Verbindung enthält, die für eine Transportschicht, als Host-Material oder als ein lichtemittierendes Material in einem lichtemittierenden Element verwendet werden kann.

Shaolong Gong et al., J. Mater. Chem. 2012, 22, 2894-2899 beschreibt Isomere von N,N'-Dicarbazolyl-4,4'-biphenyl mit hohen Triplett-Energien für effiziente blaue Elektrophosphoreszenz.

Der Artikel N. R. Madadi et al., Bioorg. Med. Chem. Lett. 2014, 24, 601-603 beschreibt die Synthese und antiproliferative Aktivität von 5-((1-Benzyl-1H-indol-3-yl)methylen)-1,3-dimethylpyrimidin-2,4,6(1H,3H,5H)-Trion-Analoga gegenüber menschlichen Tumorzelllinien.

## Beschreibung

Die Erfindung stellt eine neue Klasse von Molekülen bereit, die genau zwei Einheiten gemäß Formel I enthalten, nämlich eine erste Einheit gemäß Formel I und eine zweite Einheit gemäß Formel I.

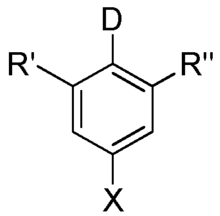

Formel I

Die beiden Einheiten gemäß Formel I sind über eine Einfachbindung miteinander verknüpft (so dass die Phenylringe über eine Einfachbindung direkt aneinander gebunden sind) oder über eine von R' und/oder R'' gebildete Brücke Y miteinander verknüpft, wobei die Einfachbindung bzw. die Brücke Y entweder über R' der ersten Einheit gemäß Formel I und R' der zweiten Einheit gemäß Formel I oder über R'' der ersten Einheit gemäß Formel I und R' der zweiten Einheit gemäß Formel I oder über R' der ersten Einheit gemäß Formel I und R'' der zweiten Einheit gemäß Formel I oder über R'' der ersten Einheit gemäß Formel I und R'' der zweiten Einheit gemäß Formel I gebildet wird. Das heißt, die Brücke Y knüpft an der Position R' und/oder R'' an bzw. die Einfachbindung besteht zwischen zwei Positionen R' und/oder R'' zweier Einheiten nach Formel I.

In Formel I ist

Y = eine beliebige divalente chemische Gruppe;
X = bei jedem Auftreten gleich oder verschieden CN oder $CF_3$;
D = chemische Einheit aufweisend eine Struktur der Formel I-1:

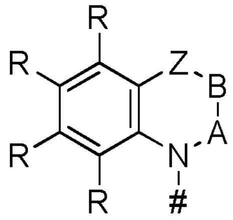

Formel I-1

wobei

# = Anknüpfungspunkt der Einheit gemäß Formel I-1 an die Struktur gemäß Formel I;
A und B = unabhängig voneinander ausgewählt aus der Gruppe bestehend aus $CRR^1$, CR, NR, N, wobei zwischen A und B eine Einfach- oder Doppelbindung vorliegt und zwischen B und Z eine Einfach- oder Doppelbindung vorliegt;
Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, $-CRR^1$, $-C=CRR^1$, -C=NR, -NR-, -O-, $-SiRR^1-$, -S-, -S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ist;
wobei jedes R und $R^1$ bei jedem Auftreten gleich oder verschieden ist H, Deuterium, Azid ($N_3^-$ ), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination dieser Systeme, oder eine quervernetzbare Einheit QE ist, die durch säurekatalytische, thermische oder UV-Quervernetzungsverfahren in Anwesenheit oder Abwesenheit eines Photoinitiators oder durch Mikrowellenstrahlung quervernetzt werden kann; dabei können zwei oder mehrere dieser Substituenten R und $R^1$ auch miteinander ein mono- oder polycyclisches, aliphatisches, aromatisches und/oder benzoannelliertes Ringsystem bilden;
$R^2$ bei jedem Auftreten gleich oder verschieden ist H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)_RR^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;
$R^3$ bei jedem Auftreten gleich oder verschieden ist H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

R' = Anknüpfungsposition für die zweite Einheit der Formel I oder ausgewählt aus der Gruppe bestehend aus Y, H, $N(R^4)_2$, $OR^4$ einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
R" = Anknüpfungsposition für die zweite Einheit der Formel I oder ausgewählt aus der Gruppe bestehend aus Y, $N(R^4)_2$, $OR^4$ einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
mit der Maßgabe, dass wenn R' gleich Y ist, R" ungleich Y ist und wenn R" gleich Y ist, R' ungleich Y ist;
und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
$R^4$ bei jedem Auftreten gleich oder verschieden ist H, Deuterium, $N(R^5)_2$, $Si(R^5)_3$, eine lineare Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;
$R^5$ bei jedem Auftreten gleich oder verschieden ist H, Deuterium, oder ein aliphatischer, aromatischer und/oder heteroaromatischer Kohlenwasserstoffrest mit 1 bis 20 C-Atomen; dabei können zwei oder mehrere Substituenten $R^5$ auch miteinander ein mono- oder polycyclisches, aliphatisches Ringsystem bilden.

In einer weiteren Ausführungsform ist R' gleich H und R" ist gleich dem Anknüpfungspunkt für die zweite Einheit der Formel I oder gleich Y.

In einer weiteren Ausführungsform ist R' ausgewählt aus der Gruppe bestehend aus Y, H, $N(R^4)_2$, $OR^4$ einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann und
R" ist ausgewählt aus der Gruppe bestehend aus Y, $N(R^4)_2$, $OR^4$ einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
mit der Maßgabe, dass wenn R' gleich Y ist, R" ungleich Y ist und wenn R" gleich Y ist, R' ungleich Y ist.

In einer Ausführungsform ist R' = Anknüpfungsposition für die zweite Einheit der Formel I oder ausgewählt aus der Gruppe bestehend aus Y, H, $N(R^4)_2$, $OR^4$, Thiophen, das mit einem oder mehreren Resten $R^4$ substituiert sein kann, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen Ringsystem mit 5 bis 60 aromatischen Ringatomen, das durch einen oder mehrere Reste $R^4$ substituiert sein kann;
und R" = Anknüpfungsposition für die zweite Einheit der Formel I oder ausgewählt aus der Gruppe bestehend aus Y, $N(R^4)_2$, $OR^4$, Thiophen, das mit einer oder mehreren Resten$R^4$ substituiert sein kann, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen Ringsystem mit 5 bis 60 aromatischen Ringatomen, das durch einen oder mehrere Reste $R^4$ substituiert sein kann;
mit der Maßgabe, dass wenn R' gleich Y ist, R" ungleich Y ist und wenn R" gleich Y ist, R' ungleich Y ist;
und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
wobei Y und $R^4$ wie oben definiert sind.

In einer Ausführungsform ist R' = Anknüpfungsposition für die zweite Einheit der Formel I oder ausgewählt aus der Gruppe bestehend aus Y, H, $N(R^4)_2$, $OR^4$, einer linearen Alkyl- oder Alkoxygruppe mit 1 bis 40 C-Atomen oder einer

verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
und R" = Anknüpfungsposition für die zweite Einheit der Formel I oder ausgewählt aus der Gruppe bestehend aus Y, $N(R^4)_2$, $OR^4$, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
mit der Maßgabe, dass das heteroaromatische Ringsystem kein N-Heteroaromat ist;
und mit der Maßgabe, dass wenn R' gleich Y ist, R" ungleich Y ist und wenn R" gleich Y ist, R' ungleich Y ist;
und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
wobei Y und $R^4$ wie oben definiert sind.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt N, O, und/oder S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen dieser Gruppen.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$ bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Die chemische Einheit D der erfindungsgemäßen Moleküle weist Donoreigenschaften auf. Was unter Donor- bzw. Akzeptoreigenschaften verstanden wird, ist dem Fachmann prinzipiell bekannt. Die chemische Einheit D ist in einer bevorzugten Ausführungsform elektronenschiebend. Sie weist einen +M-Effekt (positiven mesomeren Effekt) auf. Insbesondere weisen geeignete Donorsubstituenten ein Atom mit einem freien Elektronenpaar auf, wie zum Beispiel ein

N-, O- oder S-Atom. Hierbei sind 5-Ring-Heteroarylgruppen mit genau einem Heteroringatom bevorzugt. An diese können auch weitere Arylgruppen ankondensiert sein. Hierbei sind insbesondere Carbazolgruppen bzw. Carbazolderivate bevorzugt. Weitere geeignete Donorsubstituenten sind Phenoxazingruppen bzw. Phenoxazinderivate. Bei Letzteren kann der Sauerstoff des Phenoxazins beispielsweise durch $-CRR^1$, $-C=CRR^1$, -C=NR, -NR-, $-SiRR^1-$, -S-, -S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ersetzt sein.

In einer bevorzugten Ausführungsform übt der elektronenziehende Rest X einen -M-Effekt (negativen mesomeren Effekt) oder einen -I-Effekt (negativen induktiven Effekt) aus. Der Rest X ist entsprechend ein Akzeptorsubstituent. Geeignete Akzeptorsubstituenten sind insbesondere Cyanogruppen oder $CF_3$.

Die erfindungsgemäßen Moleküle weisen in ortho-Stellung zum Donor am Aromaten einen Substituenten auf. Dies ermöglicht eine effektive Trennung von HOMO und LUMO des organischen Moleküls.

Die erfindungsgemäßen Moleküle zeigen thermisch aktivierte verzögerte Fluoreszenz und emittieren bevorzugt im tiefblauen Bereich des sichtbaren Spektrums.

Die Verwendung der erfindungsgemäßen Moleküle in einer optoelektronischen Vorrichtung, beispielsweise einer OLED, führt zu höheren Effizienzen der Vorrichtung. Weiterhin lassen sich OLEDs im tiefblauen Farbspektrum realisieren. Entsprechende OLEDs weisen eine höhere Stabilität als OLEDs mit bekannten Emittermaterialien und vergleichbarer Farbe.

Bei den quervernetzbaren Einheiten QE handelt es sich in einer Ausführungsform um eine Verbindung ausgewählt aus der Gruppe bestehend aus Oxetanen, Alkinen und Aziden, insbesondere für eine Clickreaktion, sowie folgenden Alkenderivaten:

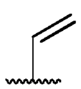
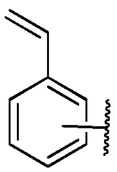
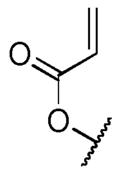
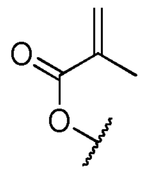
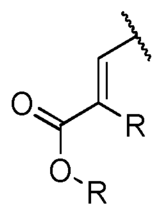
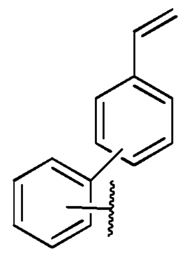

In einer alternativen Ausführungsform handelt es sich bei Z um eine kovalente Einfachbindung oder eine divalente organische Brücke ausgewählt aus substituierten und unsubstituierten Alkylen- (auch verzweigt oder zyklisch), Alkenylen-, Alkinylen-, Arylen- und Heteroarylen-Gruppen, O, NR, $C=CR_2$, C=NR, $SiR_2$ S, S(O), $S(O)_2$, BR, PR, P(O)R, wobei auch Kombinationen dieser Einheiten möglich sind (z.B. durch O unterbrochene Alkylen- (auch verzweigt oder zyklisch), Alkenylen-, Alkinylen-, Arylen- und Heteroarylen-Gruppen).

In einer bevorzugten Ausführungsform ist D unabhängig voneinander jeweils eine Donorgruppe mit elektronenschiebenden Eigenschaften, die ausgewählt aus der Gruppe bestehend aus substituierten und unsubstituierten Carbazol, substituierten und unsubstituierten Indol, substituierten und unsubstituierten Indolin, substituierten und unsubstituierten Dihydroacridin, substituierten und unsubstituierten Benzimidazol, substituierten und unsubstituierten 2,3,4,9-Tetrahydrocarbazol, substituierten und unsubstituierten 1,2,3,4-Tetrahydrochinolin, substituierten und unsubstituierten Phenothiazin, substituierten und unsubstituierten Phenoxazin, substituierten und unsubstituierten Dihydrophenazin, substituierten und unsubstituierten Spiroverbindungen.

In einer Ausführungsform des organischen Moleküls weist die Donorgruppe mit elektronenschiebenden Eigenschaften der Formel I-1 eine Struktur der Formel II auf:

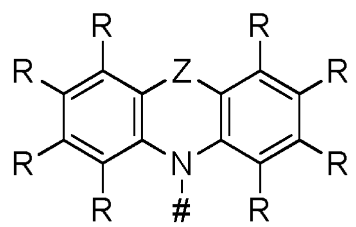

Formel II

wobei für #, Z und R die oben in Verbindung mit Formel I genannten Definitionen gelten.

Die Donorgruppe mit elektronenschiebenden Eigenschaften der Formel I-1 kann in einer Ausführungsform eine Struktur der Formel III aufweisen:

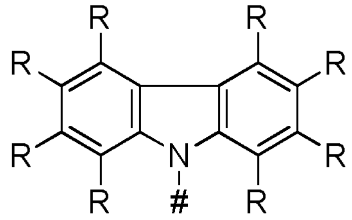

Formel III

wobei für # und R die oben in Verbindung mit Formel I genannten Definitionen gelten.

Beispiele für erfindungsgemäße Donoren:

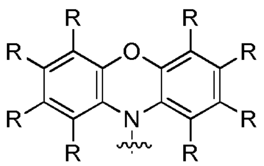
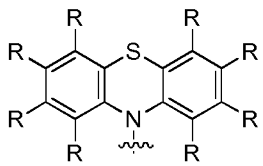
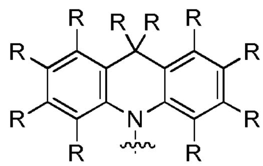
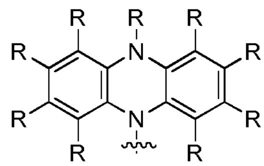

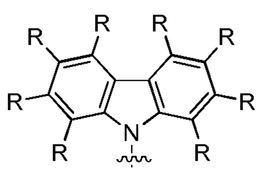
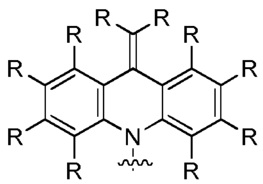
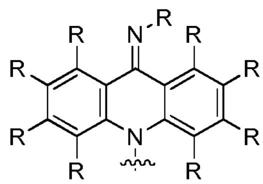
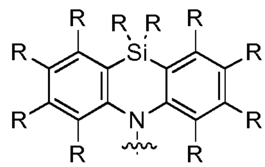

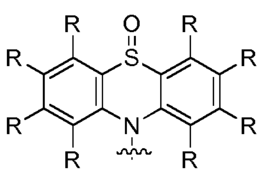
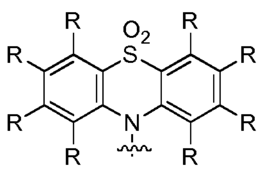
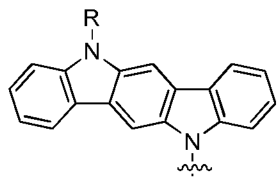
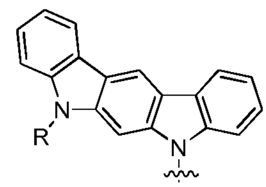

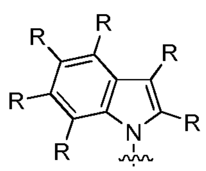
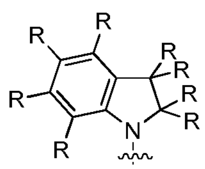
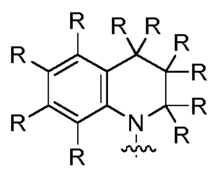
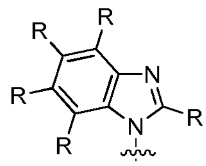

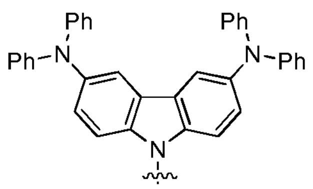
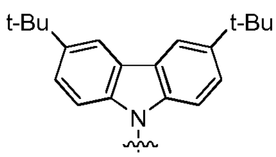
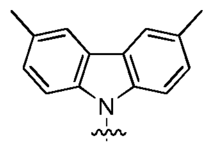
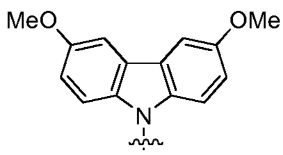

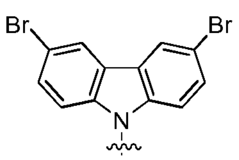
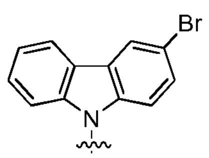
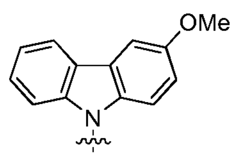
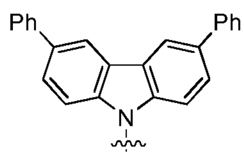

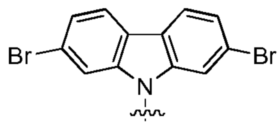
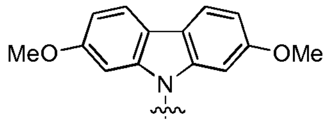
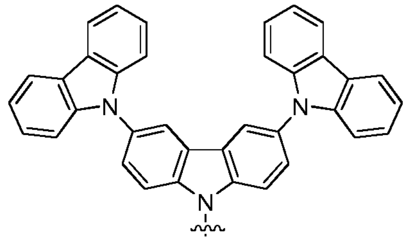

Die akzeptierende Einheit X der Formel I ist in einer Ausführungsform gleich CN, in einer weiteren Ausführungsform gleich $CF_3$. In einer weiteren Ausführungsform der Erfindung ist der Rest R' der Formel I ein Wasserstoffatom, also H.

Die divalente Gruppe Y ist eine organische Brücke, die in einer Ausführungsform eine substituierte oder unsubstituierte Alkylen-, Alkenylen-, Alkinylen-, Arylen- oder Heteroarylen-Gruppe oder eine Kombination dieser, oder -O-, -NR-, -C=$CR_2$, -C=NR, -$SiR_2$- -S-, -S(O)-, - $S(O)_2$-, durch O unterbrochene Alkyl- (auch verzweigt oder zyklisch), Heteroalkyl-, Aryl-, Heteroaryl-, Alkenyl-Gruppen, Phenyl- und substituierte Phenyleinheiten ist; wobei für R die oben genannten Definitionen gelten.

Die Brücke Y kann beispielsweise ausgewählt sein aus einer der Strukturen der Formeln IV bis X:

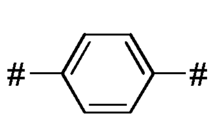
IV

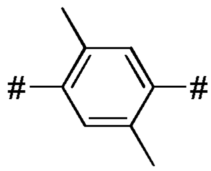
V

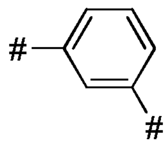
VI

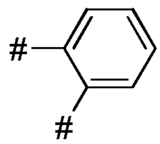
VII

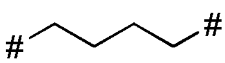
VIII

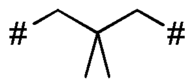
IX

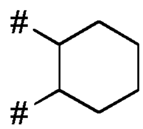
X

mit
# = Anknüpfungspunkt der Brücke Y an die Struktur gemäß Formel I (also an die mit R' und/oder R" gekennzeichneten Positionen der ersten bzw. zweiten Einheit der Formel I).

In bestimmten Ausführungsformen weisen die erfindungsgemäßen Moleküle eine Struktur der Formel XI-a, XI-b oder XI-c auf:

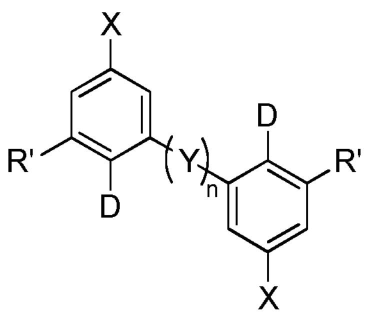
Formel XI-a

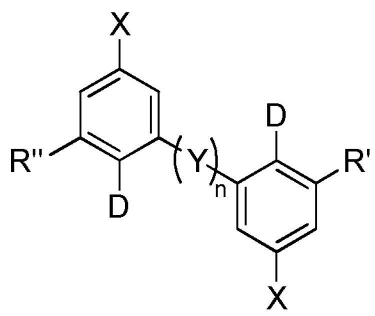
Formel XI-b

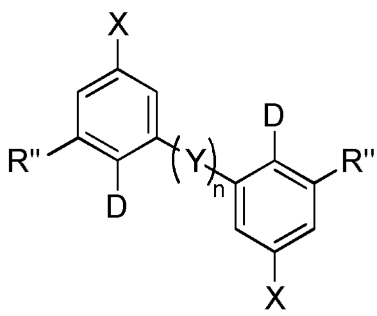
Formel XI-c

mit

n = 0 oder 1;
und wobei für D, R', R", X und Y die oben genannten Definitionen gelten.

In einer Ausführungsform weisen die erfindungsgemäßen Moleküle eine Struktur der Formel XII auf.

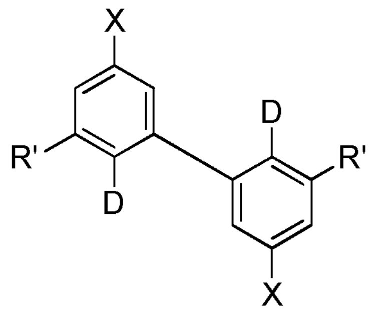

Formel XII

und wobei für D, R' und X die oben genannten Definitionen gelten.

In einer noch bevorzugteren Ausführungsform weisen die erfindungsgemäßen Moleküle eine Struktur der Formel XIII auf:

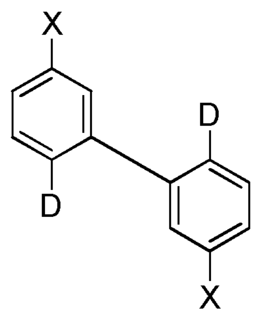

Formel XIII

und wobei für D und X die oben genannten Definitionen gelten.

In einem weiteren Aspekt betrifft die Erfindung ein Verfahren zur Herstellung eines erfindungsgemäßen organischen Moleküls der hier beschriebenen Arten.

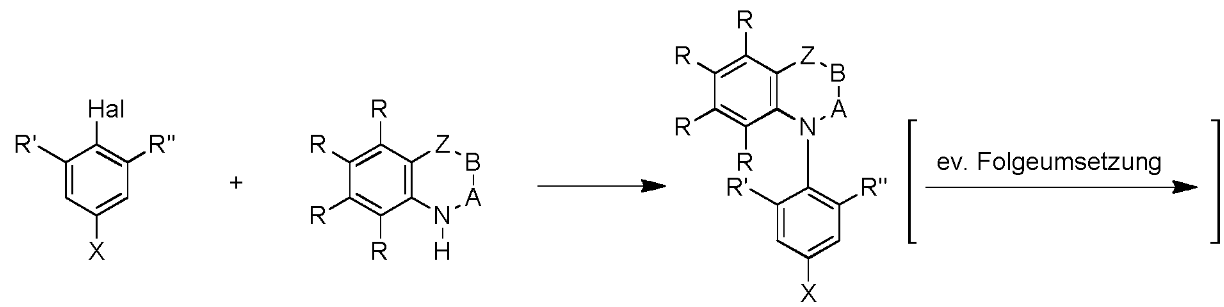


Gezeigt ist die Synthese für die erfindungsgemäßen Moleküle mit einer Einfachbindung zwischen den Phenylringen und für eine Bindung über eine Brücke Y, jeweils mit mindestens einer optionalen Folgeumsetzung.

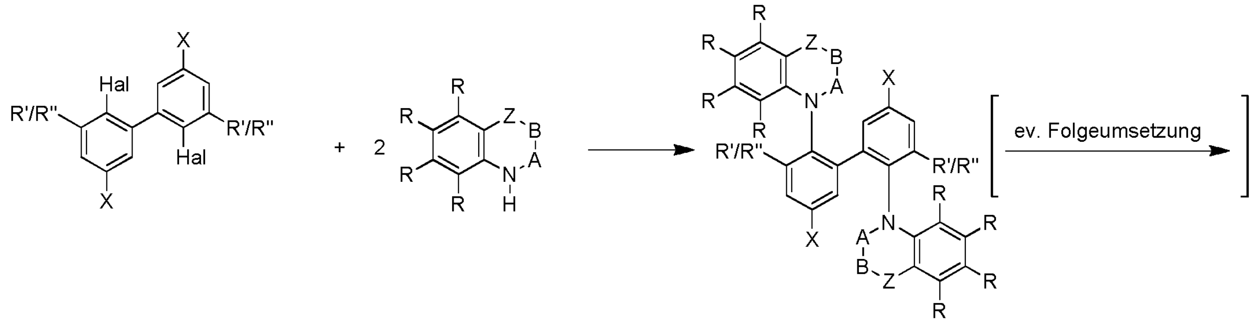


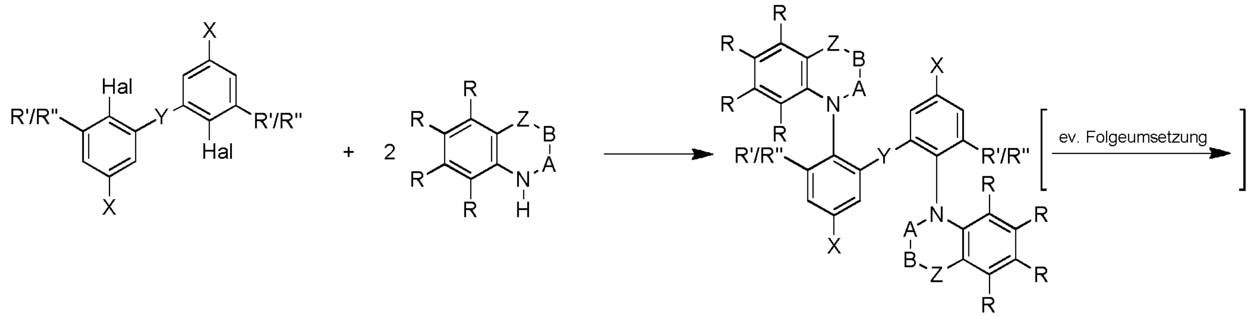


Hierbei wird ein Stickstoffheterozyklus im Sinne einer nukleophilen aromatischen Substitution mit einem Arylhalogenid, bevorzugt einem Arylfluorid umgesetzt. Typische Bedingungen beinhalten die Verwendung einer Base, wie beispielweise tribasisches Kaliumphosphat oder Natriumhydrid in einem aprotisch polarem Lösungsmittel, wie beispielweise Dimetylsulfoxid (DMSO) oder N,N-Dimethylformamid (DMF).

Eine alternative Syntheseroute beinhaltet die Einführung eines Stickstoffheterozyklus über eine Kupfer- oder Palladium-katalysierte Kupplung mit einem Arylhalogenid oder Arylpseudohalogenid, bevorzugt ein Arylbromid, ein Aryliodid, Aryltriflat oder ein Aryltosylat. Die beschriebenen Herstellungsweisen können dabei sowohl die letzte synthetische Umsetzung darstellen als auch ein Vorläufermolekül liefern, welches durch Folgeumsetzungen, beispielsweise durch Änderung der Reste R, R' bzw. R", zum erfindungsgemäßen Molekül umgesetzt werden kann.

Ein weiterer Aspekt der Erfindung betrifft die Verwendung eines organischen Moleküls der hier beschriebenen Art als lumineszierender Emitter oder als Absorber, und/oder als Hostmaterial und/oder als Elektronentransportmaterial, und/oder als Lochinjektionsmaterial, und/oder als Lochblockiermaterial in einer organischen optoelektronischen Vorrichtung.

Im Rahmen einer derartigen Verwendung ist die organische optoelektronische Vorrichtung insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Bei der Verwendung beträgt der Anteil des organischen Moleküls an der Emissionsschicht in einer organischen optoelektronischen Vorrichtung, insbesondere in OLEDs, 1 % bis 99 %, insbesondere 5 % bis 80 % (Gew%). In einer alternativen Ausführungsform beträgt der Anteil des organischen Moleküls an der Emissionsschicht 100%.

In einer Ausführungsform weist die lichtemittierende Schicht neben dem erfindungsgemäßen organischen Molekül ein Hostmaterial auf, dessen Triplett (T1)- und Singulett (S1)-Energieniveaus energetisch höher liegen als die Triplett (T1)- und Singulet (S1)-Energieniveaus des organischen Moleküls.

In einem weiteren Aspekt betrifft die Erfindung eine organische optoelektronische Vorrichtung, aufweisend ein organisches Molekül der hier beschriebenen Art, insbesondere ausgeformt als eine Vorrichtung ausgewählt aus der

Gruppe bestehend aus organischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine derartige organische optoelektronische Vorrichtung weist in einer Ausführungsform auf:

- ein Substrat,
- eine Anode und
- eine Kathode, wobei insbesondere die Anode oder die Kathode unmittelbar auf das Substrat aufgebracht sind, und
- mindestens eine lichtemittierende Schicht, die zwischen Anode und Kathode angeordnet ist und die das erfindungsgemäße organische Molekül aufweist.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier und im Folgenden wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)

2. Kathode

3. Elektroneninjektionsschicht (electron injection layer, EIL)

4. Elektronenleitschicht (electron transport layer, ETL)

5. Lochblockierschicht (hole blocking layer, HBL)

6. Emissionsschicht bzw. Emitterschicht (emitting layer, EML)

7. Elektronenblockierschicht (electron blocking layer, EBL)

8. Lochtransportschicht (hole transport layer, HTL)

9. Lochinjektionsschicht (hole injection layer, HIL)

10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselungsanordnung angeordnet sein. Die Verkapselungsanordnung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, Siliziumwafer oder jedes andere geeignete feste oder flexible, optional durchsichtige Material dienen.
Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4''-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4''-Tris[2-naphthyl(phenyl)amino]triphenylamin), Spiro-TAD, DNTPD, NPNPB, MeO-TPD, HAT-CN oder TrisPcz (9,9'-Diphenyl-6-(9-phenyl-9H-carbazol-3-yl)-9H,9'H-3,3'-bicarbazol) dienen.
Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen.

Die Emitter-Schicht EML enthält oder besteht aus Emittermaterial oder einer Mischung aufweisend mindestens zwei Emittermaterialien und optional aus einem oder mehreren Hostmaterialien. Geeignete Hostmaterialien sind beispielsweise mCP, TCTA, 2-TNATA, mCBP, Sif87 (Dibenzo[b,d]thiophen-2-yltriphenylsilan), Sif88 (Dibenzo[b,d]thiophen-2-yl)diphenylsilan), CBP (4,4'-Bis-(N-carbazolyl)-biphenyl) oder DPEPO (Bis[2-((oxo)diphenylphosphino)phenyl]ether). Für im Grünen oder im Roten emittierendes Emittermaterial oder einer Mischung aufweisend mindestens

zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen.

Die Elektronentransportschicht ETL kann beispielsweise Materialien auf Basis von $AlQ_3$, TSPO1, BPyTP2 (2,7-Di(2,2'-bipyridin-5-yl)triphenyl) oder BTB (4,4'-Bis-[2-(4,6-diphenyl-1,3,5-triazinyl)]-1,1'-biphenyl) aufweisen.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100-200 nm. Besonders bevorzugt werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.
Dem Fachmann ist hierbei bekannt, welche Kombinationen der Materialien für eine optoelektronische Vorrichtung enthaltend ein erfindungsgemäßes organisches Molekül zu nutzen sind.

In einer Ausführungsform der erfindungsgemäßen organischen optoelektronischen Vorrichtung ist das erfindungsgemäße organische Molekül als Emissionsmaterial in einer lichtemittierenden Schicht eingesetzt, wobei es entweder als Reinschicht oder in Kombination mit einem Matrixmaterial eingesetzt ist.

Der Anteil des erfindungsgemäßen organischen Moleküls an der Emissionsschicht beträgt in einer weiteren Ausführungsform in einer lichtemittierenden Schicht in optischen Licht emittierenden Vorrichtungen, insbesondere in OLEDs, zwischen 5% und 80%. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierende Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein Hostmaterial aufweist, dessen Triplett (T1)- und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

Erfindungsgemäß ist auch ein lichtemittierendes Material, aufweisend ein erfindungsgemäßes organisches Molekül und ein Hostmaterial, wobei die Triplett (T1)- und Singulett (S1)-Energieniveaus des Hostmaterials energetisch höher liegen als die Triplett (T1)- und Singulett (S1)-Energieniveaus des organischen Moleküls, und wobei das lichtemittierende Material Fluoreszenz oder thermisch aktivierte verzögerte Fluoreszenz emittiert, und einen deltaE(S1-T1)-Wert zwischen dem untersten angeregten Singulett (S1)- und dem darunter liegenden Triplett (T1)-Zustand von kleiner als 3000 $cm^{-1}$ aufweist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,

- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

## Beispiele

**Allgemeine Arbeitsvorschriften: Photophysikalische Messungen *Vorbehandlung von optischen Gläsern***

Nach jeder Benutzung wurden die optischen Gläser (Küvetten und Substrate aus Quarzglas, Durchmesser: 1 cm) gereinigt. Je dreimaliges Spülen mit Dichlormethan, Aceton, Ethanol, demineralisiertem Wasser. Einlegen in 5 % Hellmanex-Lösung für 24 h, gründliches Ausspülen mit demineralisiertem Wasser, zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.

***Probenvorbereitung, Film: Spin-Coating***

Gerät: Spin150, SPS euro.

Die Probenkonzentration entsprach 10mg/ml, angesetzt in Toluol oder Chlorbenzol. Programm: 1) 3 s bei 400 U/min; 2) 20 Sek. bei 1000 U/min bei 1000 Upm/ s. 3) 10s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen:

UV-VIS-Spektren wurden auf einem Gerät der Firma Thermo Scientific, Modell Evolution 201 aufgenommen. (Siehe Probenvorbereitung: Lösungen)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

***Quanteneffizienzbestimmung***

Die Messung der Photolumineszenzquantenausbeute erfolgte mittels eines *Absolute PL Quantum Yield Measurement* C9920-03G-Systems der Firma *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute $\Phi$ in % und die CIE-Farbkoordinaten als x,y-Werte angegeben.

PLQY wurde für Polymerfilme, Lösungen und Pulverproben nach folgendem Protokoll bestimmt:
Als Referenzmaterial dient Anthracene in Ethanol mit bekannter Konzentration. Es wurde zuerst das Absorbtionsmaximum der Probe bestimmt und mit diesem angeregt. Anschließend wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Berechnungen nach der Dichtefuntionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F.; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, eine Entwicklung der Universität Karlsruhe und Forschungszentrum Karlsruhe GmbH, 1989-2007, TURBOMOLE GmbH, seit 2007; http://www.turbomole.com) durchgeführt.

**Beispiel** 1

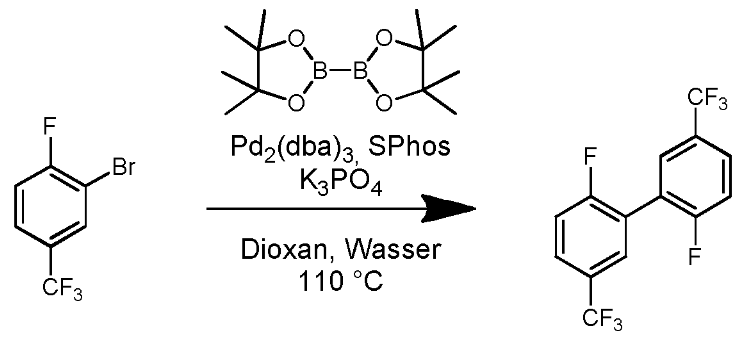


**1a**

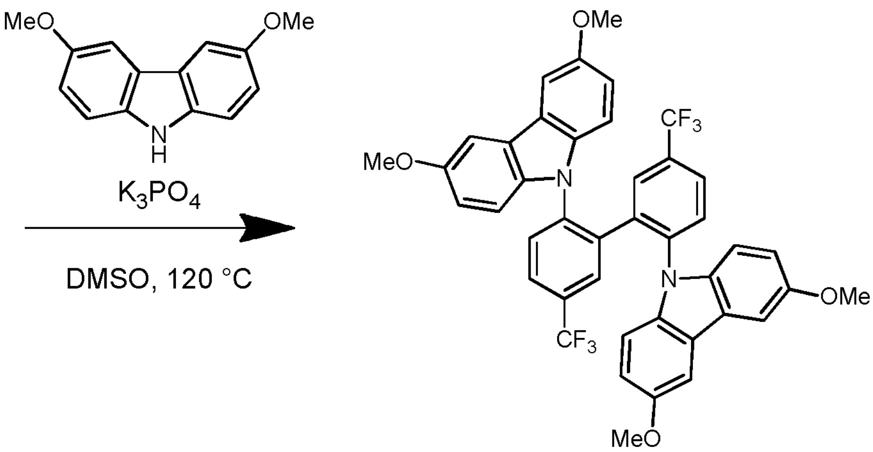


**1**

Stufe 1:

3-Brom-4-flruorbenzotrifluorid (65,8 mmol), Bis(pinacolato)diboron (32,9 mmol), $Pd_2(dba)_3$ (0,33 mmol), SPhos (1,32 mmol) und tribasisches Kaliumphosphat (197 mmol) werden unter Stickstoff in einem Dioxan/Wasser-Gemisch (300 mL / 20 mL) bei 110 °C für 16 h gerührt. Anschließend werden die unlöslichen Bestandteile der Reaktionsmischung abfiltriert und mit Dioxan nachgewaschen. Das Lösemittel des Filtrats wird entfernt und der erhaltene Rückstand in Dichlormethan gelöst und über wenig Kiesegel filtriert. Das Produkt wird als gelber Feststoff erhalten. $^1$H NMR (500 MHz, Chloroform-*d*) $\delta$ = 7.72 - 7.69 (m, 4H), 7.34 - 7.31 (m, 2H) ppm.

Stufe 2:

**1a** (5 mmol), 3,6-Dimethoxycarbazol (12 mmol) und tribasisches Kaliumphosphat (20 mmol) werden unter Stickstoff in DMSO (20 mL) suspendiert und bei 100 °C gerührt (16 h). Anschließend wird die Reaktionsmischung in 150 mL Wasser gegeben und mit Dichlormethan (2 x 100 mL) extrahiert. Die vereinigten organischen Phasen werden mit gesättigter Natriumchlorid-Lösung gewaschen (2 x 150 mL), getrocknet über Magnesiumsulfat und das Lösemittel anschließend entfernt. Das Rohprodukt wurde in Dichlormethan aufgenommen und über wenig Kieselgel filtriert (Eluent: Dichlormethan). Nach entfernen des Lösemittels wurde schließlich durch Umkristallisation aus einer Mischung von Ethanol und Chloroform gereinigt. Das Produkt **1** wurde als weißer Feststoff erhalten.
$^1$H NMR (500 MHz, Chloroform-d) $\delta$ 8.15 (d, J = 2.1 Hz, 2H), 7.59 (dd, J = 8.3, 2.1 Hz, 2H), 7.23 (d, J = 8.3 Hz, 3H), 6.94 (br s, 2H), 6.79-6.48 (br m, 4H), 6.36 (br s, 2H), 5.68 (br s, 2H), 3.86 (s, 12H).
Die Filmemission von **1** (10 % in PMMA) ist Figur 1 zu entnehmen. Das Emissionsmaximum liegt bei 461 nm. Die Photolumineszenzquantenausbeute (PLQY) beträgt 47%.

**Beispiel 2**

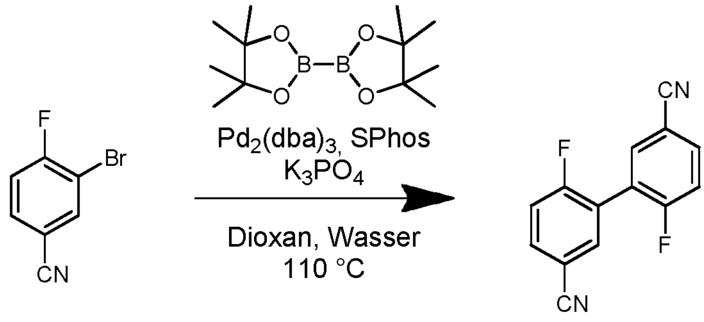


**2a**

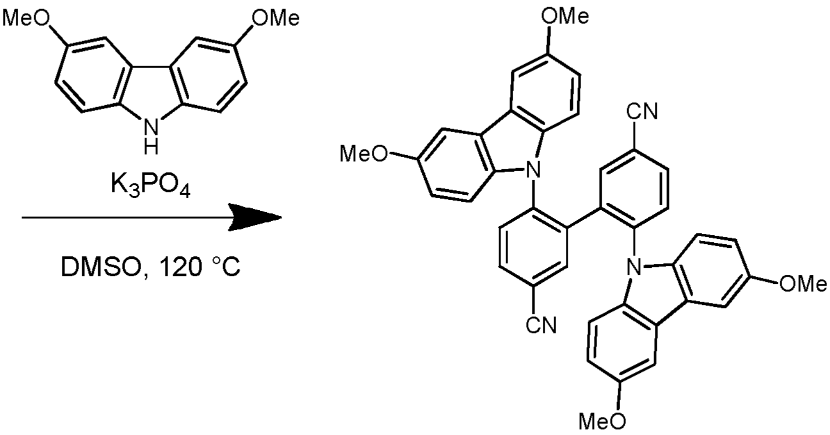


**2**

Stufe 1:

3-Brom-4-fluorbenzonitril (125 mmol), Bis(pinacolato)diboron (62,5 mmol), $Pd_2(dba)_3$ (0,63 mmol), SPhos (2,50 mmol) und tribasisches Kaliumphosphat (375 mmol) werden unter Stickstoff in einem Dioxan/Wasser-Gemisch (350 mL / 20 mL) bei 110 °C für 16 h gerührt.

Anschließend werden die unlöslichen Bestandteile der Reaktionsmischung abfiltriert und mit Dioxan nachgewaschen. Das Lösemittel des Filtrats wird entfernt und der erhaltene Rückstand in Tetrahydrofuran gelöst und über wenig Kieselgel filtriert. Das Produkt wird als gelber Feststoff erhalten. $^1H$ NMR (500 MHz, Chloroform-*d*) $\delta$ = 7.79 - 7.76 (m, 2H), 7.74 - 7.73 (m, 2H), 7.36 - 7.33 (m, 2H) ppm.

Stufe 2:

**2a** (8.33 mmol), 3,6-Dimethoxycarbazol (19,2 mmol) und tribasisches Kaliumphosphat (33,3 mmol) werden unter Stickstoff in DMSO (30 mL) suspendiert und bei 110 °C gerührt (16 h). Anschließend wird die Reaktionsmischung in 400 mL gesättigte Natriumchlorid-Lösung gegeben und mit Dichlormethan (3 x 150 mL) extrahiert. Die vereinigten organischen Phasen werden mit gesättigter Natriumchlorid-Lösung gewaschen (2 x 150 mL), getrocknet über Magnesiumsulfat und das Lösemittel anschließend entfernt. Das Rohprodukt wurde schließlich durch Umkristallisation aus Toluol gereinigt. Das Produkt 2 wurde als gelber Feststoff erhalten.
$^1H$ NMR (500 MHz, Chloroform-*d*) $\delta$ = 8.19 (d, J = 1.9 Hz, 2H), 7.62 (dd, J = 8.3, 2.0 Hz, 2H), 7.24 (d, J = 8.3 Hz, 2H), 7.21 (br s, 2H), 6.87 (br s, 2H), 6.72 (br s, 2H), 6.49 (br s, 2H), 6.38 (br s, 2H), 5.65 (br s, 2H), 3.94 - 3.75 (m, 12H).

Die Filmemission von **2** (10 % in PMMA) ist Figur 2 zu entnehmen. Das Emissionsmaximum liegt bei 478 nm.

Die Photolumineszenzquantenausbeute (PLQY) beträgt 75%. Die Emissionslebensdauer beträgt 25 µs.

**Beispiel 3**

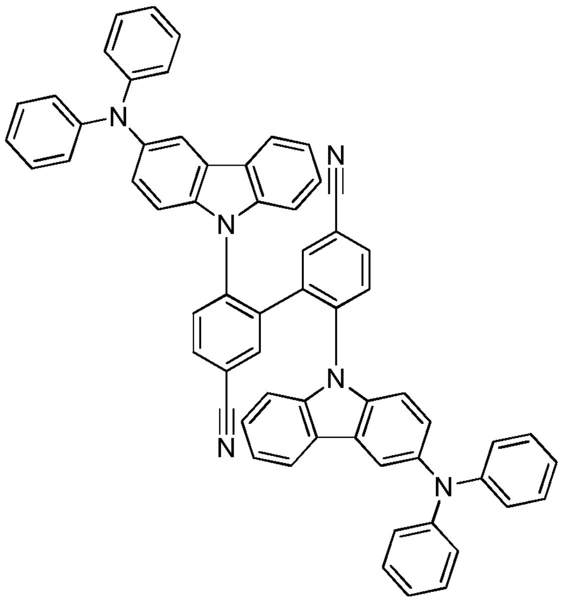

**3**

Dünnschichtchromatografie: $R_f$ = 0,5 (Cyclohexan/Ethylacetat 5:1)

Die Filmemission von **3** (10 % in PMMA) ist Figur 3 zu entnehmen. Das Emissionsmaximum liegt bei 495 nm. Die Photolumineszenzquantenausbeute (PLQY) beträgt 64%. Die Emissionslebensdauer beträgt 13 µs.

**Beispiel 4**

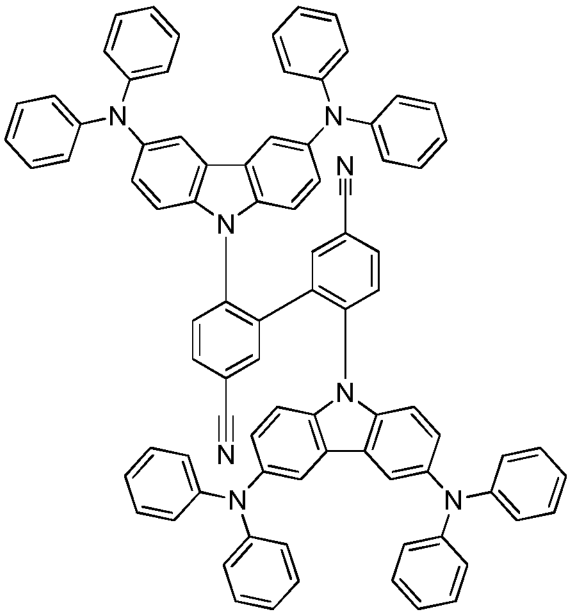

**4**

Dünnschichtchromatografie: $R_f$ = 0,5 (Cyclohexan/Ethylacetat 5:1)

Die Filmemission von **4** (10 % in PMMA) ist Figur 4 zu entnehmen. Das Emissionsmaximum liegt bei 507 nm. Die Photolumineszenzquantenausbeute (PLQY) beträgt 66%. Die Emissionslebensdauer beträgt 10 µs.

**Beispiel 5**

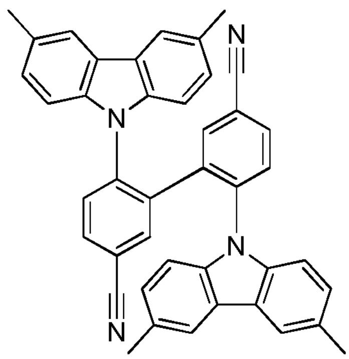

**5**

Dünnschichtchromatografie: $R_f$ = 0,5 (Cyclohexan/Ethylacetat 5:1)

Die Filmemission von **5** (10 % in PMMA) ist Figur 5 zu entnehmen. Das Emissionsmaximum liegt bei 450 nm. Die Photolumineszenzquantenausbeute (PLQY) beträgt 62 % und die Halbwertsbreite (FWHM) beträgt 64 nm.

**Beispiel 6**

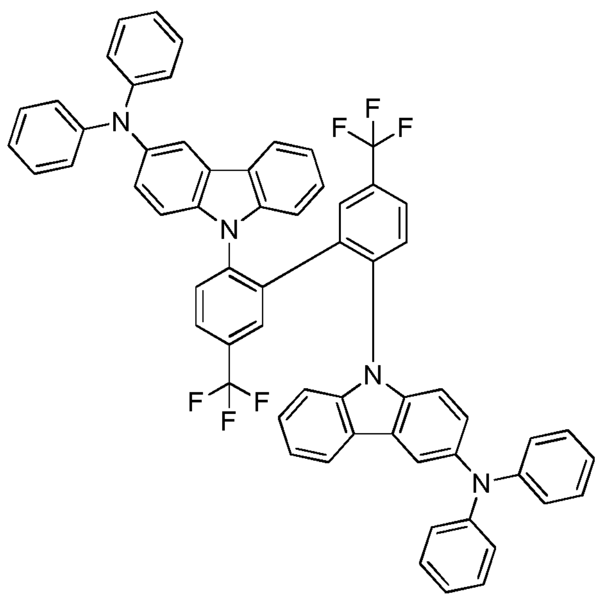

**6**

Dünnschichtchromatografie: $R_f$ = 0,6 (Cyclohexan/Ethylacetat 10:1)

Die Filmemission von **6** (10 % in PMMA) ist Figur 6 zu entnehmen. Das Emissionsmaximum liegt bei 472 nm. Die Photolumineszenzquantenausbeute (PLQY) beträgt 32 %.

**Beispiel 7**

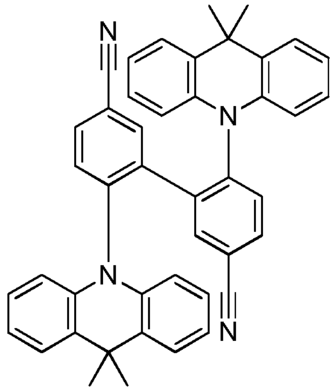

**16**

$^1$H NMR (500 MHz, Chloroform-d) δ 7.79 (dd, J = 8.2, 2.0 Hz, 2H), 7.63 (d, J = 2.0 Hz, 2H), 7.48 - 7.32 (m, 6H), 7.20-5.30 (br m, J = 510.3 Hz, 12H), 1.87 (s, 6H), 0.80 (s, 6H).

Die Filmemission von **7** (10 % in PMMA) ist Figur 7 zu entnehmen. Das Emissionsmaximum liegt bei 471 nm. Die Photolumineszenzquantenausbeute (PLQY) beträgt 65% und die Halbwertsbreite (FWHM) beträgt 83 nm. Die Emissionslebensdauer beträgt 21 µs.

**Beispiel 8**

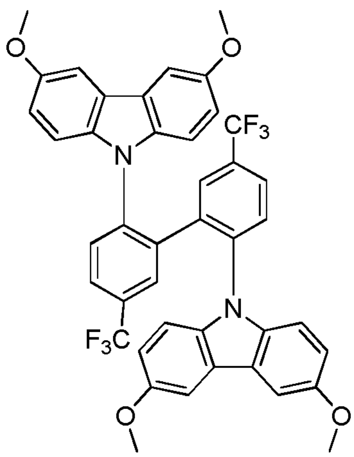

**8**

Die Filmemission von **8** (10 % in PMMA) wurde gemessen. Das Emissionsmaximum liegt bei 461 nm. Die Photolumineszenzquantenausbeute (PLQY) beträgt 47 %. Die Halbwertsbreite (FWHM) beträgt 76 nm.

**Beispiel 9**

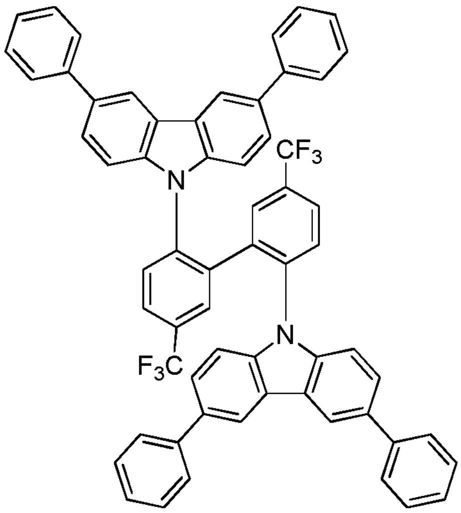

**9**

Die Filmemission von **9** (10 % in PMMA) ist Figur 9 zu entnehmen. Das Emissionsmaximum liegt bei 430 nm. Die Photolumineszenzquantenausbeute (PLQY) beträgt 23 %. Die Halbwertsbreite (FWHM) beträgt 76 nm.

**Beispiel 10**

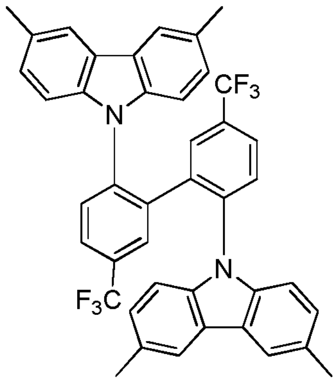

**10**

Die Filmemission von **10** (10 % in PMMA) ist Figur 10 zu entnehmen. Das Emissionsmaximum liegt bei 434 nm. Die Photolumineszenzquantenausbeute (PLQY) beträgt 30 %. Die Halbwertsbreite (FWHM) beträgt 67 nm.

**Beispiel 11**

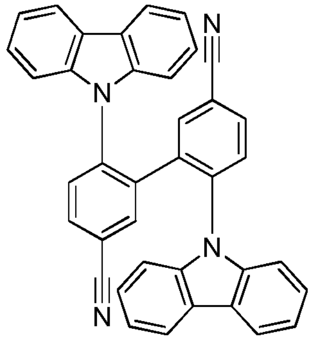

**11**

Die Filmemission von **11** (10 % in PMMA) ist Figur 11 zu entnehmen. Das Emissionsmaximum liegt bei 432 nm. Die Photolumineszenzquantenausbeute (PLQY) beträgt 42 %. Die Halbwertsbreite (FWHM) beträgt 62 nm.

**Beispiel 12**

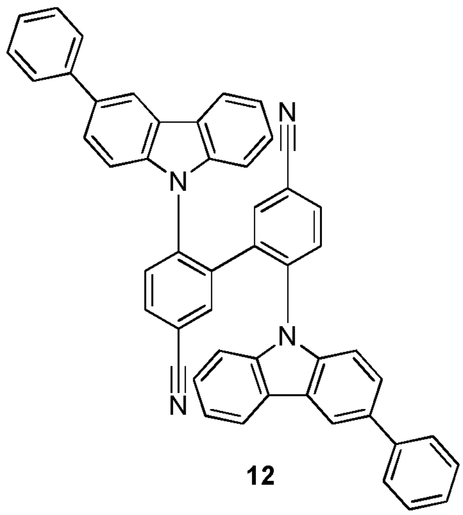

**12**

Die Filmemission von **12** (10 % in PMMA) wurde gemessen. Das Emissionsmaximum liegt bei 441 nm. Die Photolumineszenzquantenausbeute (PLQY) beträgt 58 %. Die Halbwertsbreite (FWHM) beträgt 61 nm.

**Beispiel 13**

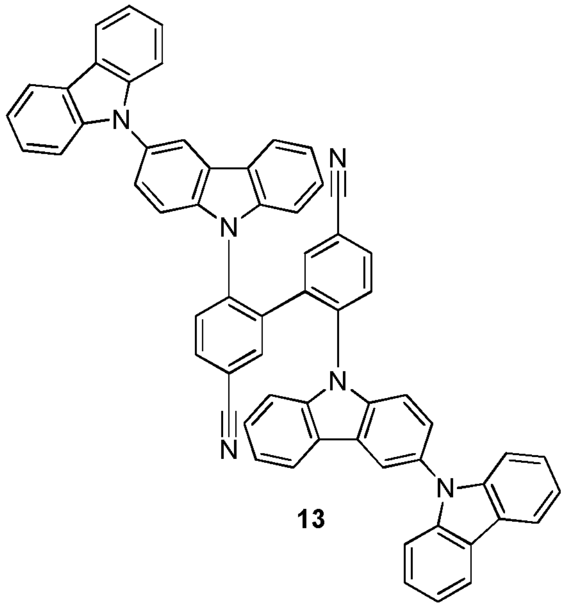

13

Die Filmemission von **13** (10 % in PMMA) wurde gemessen. Das Emissionsmaximum liegt bei 441 nm. Die Photolumineszenzquantenausbeute (PLQY) beträgt 48 %. Die Halbwertsbreite (FWHM) beträgt 66 nm.

**Beispiel 14**

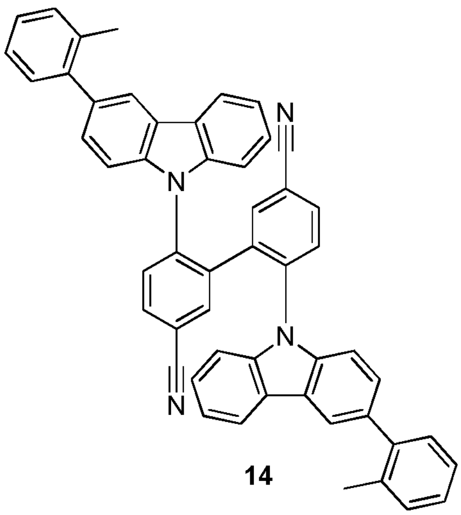

14

Die Filmemission von **14** (10 % in PMMA) wurde gemessen. Das Emissionsmaximum liegt bei 433 nm. Die Photolumineszenzquantenausbeute (PLQY) beträgt 53 %. Die Halbwertsbreite (FWHM) beträgt 59 nm.

**Beispiel 15**

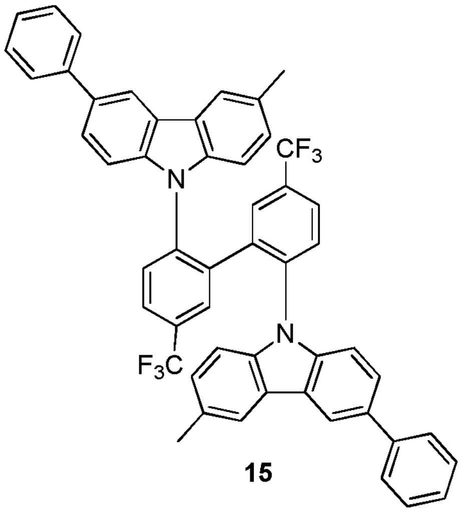

15

Die Filmemission von **15** (10 % in PMMA) wurde gemessen. Das Emissionsmaximum liegt bei 431 nm. Die Photolumineszenzquantenausbeute (PLQY) beträgt 21 %. Die Halbwertsbreite (FWHM) beträgt 68 nm.

**Beispiel 16**

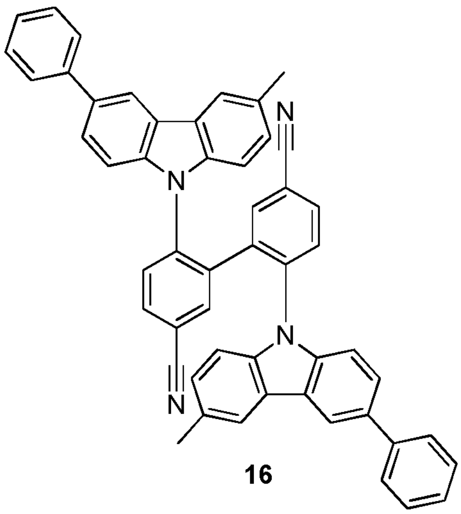

16

Die Filmemission von **16** (10 % in PMMA) wurde gemessen. Das Emissionsmaximum liegt bei 448 nm. Die Photolumineszenzquantenausbeute (PLQY) beträgt 50 %. Die Halbwertsbreite (FWHM) beträgt 62 nm.

**Beispiel 17**

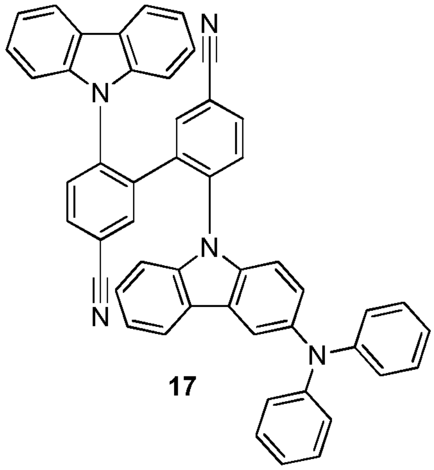

**17**

Die Filmemission von **17** (10 % in PMMA) wurde gemessen. Das Emissionsmaximum liegt bei 486 nm. Die Photolumineszenzquantenausbeute (PLQY) beträgt 61 %. Die Halbwertsbreite (FWHM) beträgt 91 nm.

**Beispiel 18**

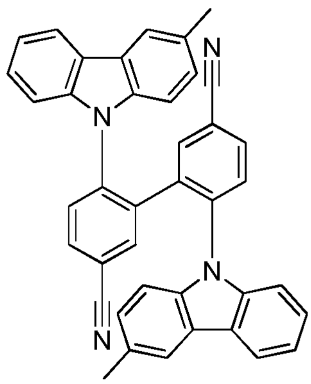

**18**

Die Filmemission von **18** (10 % in PMMA) wurde gemessen. Das Emissionsmaximum liegt bei 442 nm. Die Photolumineszenzquantenausbeute (PLQY) beträgt 56 %. Die Halbwertsbreite (FWHM) beträgt 62 nm.

**Beispiel 19**

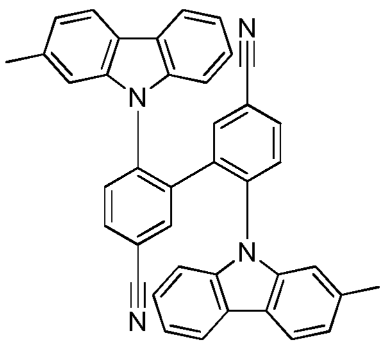

**19**

Die Filmemission von **19** (10 % in PMMA) wurde gemessen. Das Emissionsmaximum liegt bei 435 nm. Die Photolumineszenzquantenausbeute (PLQY) beträgt 45 %. Die Halbwertsbreite (FWHM) beträgt 58 nm.

**Beispiel 20**

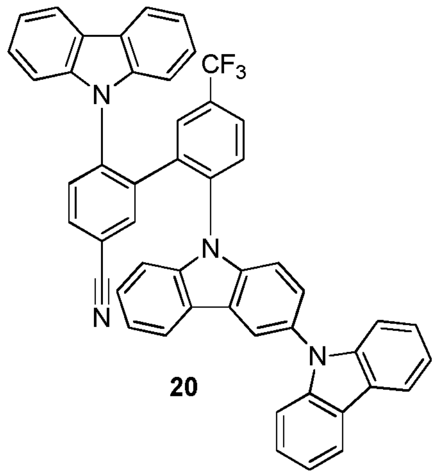

**20**

Die Filmemission von **20** wurde gemessen. Das Emissionsmaximum liegt bei 425 nm. Die Photolumineszenzquantenausbeute (PLQY) beträgt 31 %. Die Halbwertsbreite (FWHM) beträgt 63 nm.

**Beispiel 21**

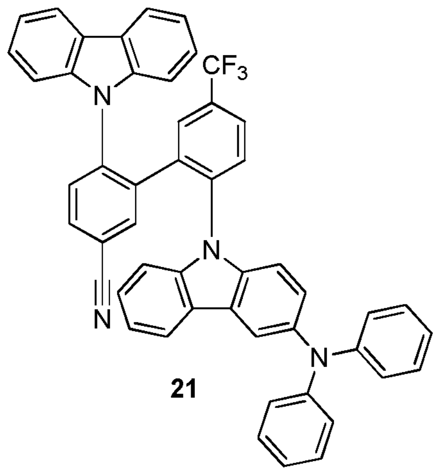

**21**

Die Filmemission von **21** (10 % in PMMA) wurde gemessen. Das Emissionsmaximum liegt bei 475 nm. Die Photolumineszenzquantenausbeute (PLQY) beträgt 39 %. Die Halbwertsbreite (FWHM) beträgt 92 nm.

**Beispiel 22**

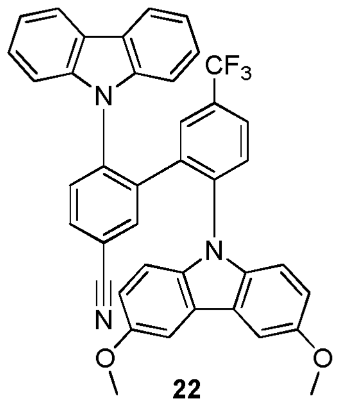

22

Die Filmemission von **22** (10 % in PMMA) wurde gemessen. Das Emissionsmaximum liegt bei 459 nm. Die Photolumineszenzquantenausbeute (PLQY) beträgt 54 %. Die Halbwertsbreite (FWHM) beträgt 74 nm.

**Beispiel 23**

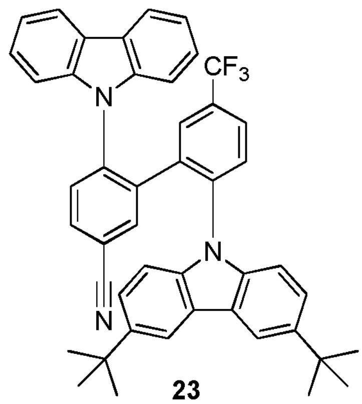

23

Die Filmemission von **23** (10 % in PMMA) wurde gemessen. Das Emissionsmaximum liegt bei 432 nm. Die Photolumineszenzquantenausbeute (PLQY) beträgt 40 %. Die Halbwertsbreite (FWHM) beträgt 61 nm.

**Beispiel 24**

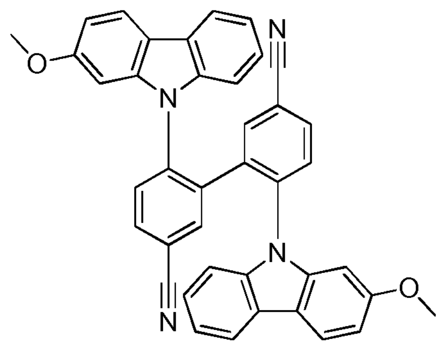

24

Die Filmemission von **24** (10 % in PMMA) wurde gemessen. Das Emissionsmaximum liegt bei 437 nm. Die Photolumineszenzquantenausbeute (PLQY) beträgt 52 %. Die Halbwertsbreite (FWHM) beträgt 61 nm.

**Beispiel 25**

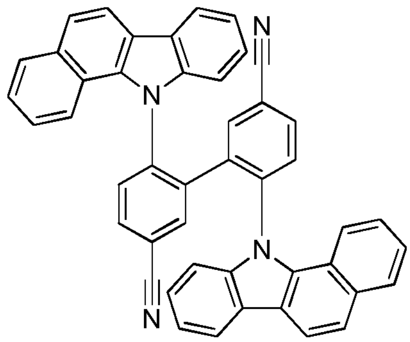

**25**

Die Filmemission von **25** (10 % in PMMA) wurde gemessen. Das Emissionsmaximum liegt bei 449 nm. Die Photolumineszenzquantenausbeute (PLQY) beträgt 25 %. Die Halbwertsbreite (FWHM) beträgt 81 nm.

**Beispiel 26**

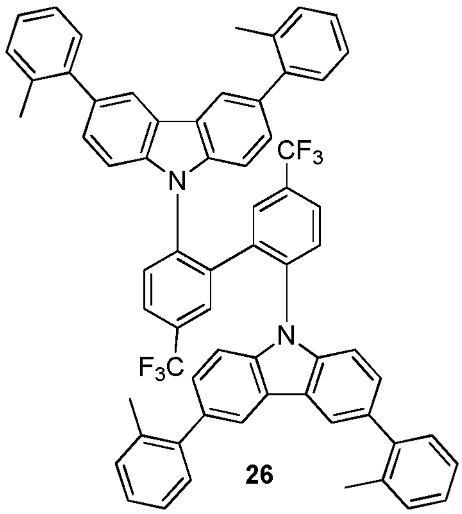

**26**

Die Filmemission von **26** (10 % in PMMA) wurde gemessen. Das Emissionsmaximum liegt bei 420 nm. Die Photolumineszenzquantenausbeute (PLQY) beträgt 22 %. Die Halbwertsbreite (FWHM) beträgt 61 nm.

**Beispiel 27**

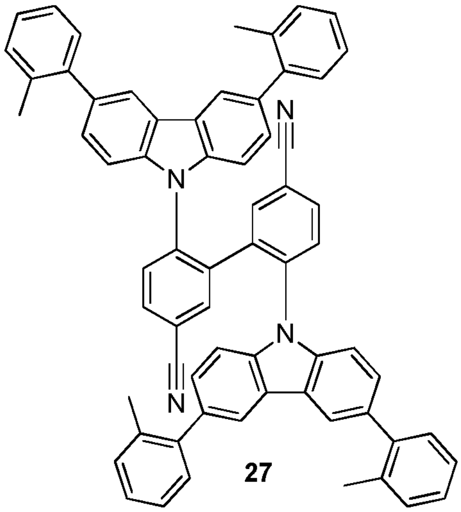

27

Die Filmemission von **27** (10 % in PMMA) ist Figur 28 zu entnehmen. Das Emissionsmaximum liegt bei 435 nm. Die Photolumineszenzquantenausbeute (PLQY) beträgt 55 %. Die Halbwertsbreite (FWHM) beträgt 58 nm.

**Beispiel 28**

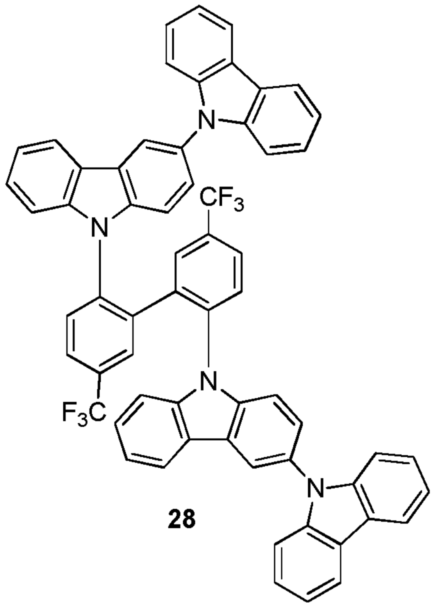

28

Die Filmemission von **28** (10 % in PMMA) wurde gemessen. Das Emissionsmaximum liegt bei 423 nm. Die Photolumineszenzquantenausbeute (PLQY) beträgt 20 %. Die Halbwertsbreite (FWHM) beträgt 71 nm.

**Beispiel 29**

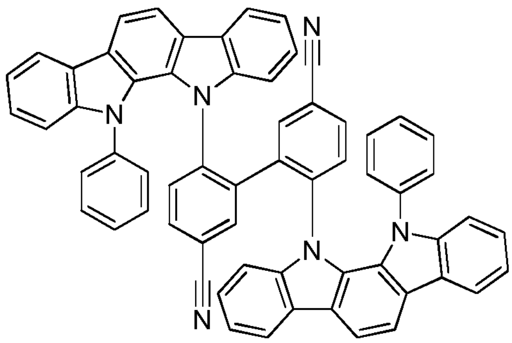

**29**

Die Filmemission von **29** (10 % in PMMA) wurde gemessen. Das Emissionsmaximum liegt bei 429 nm. Die Photolumineszenzquantenausbeute (PLQY) beträgt 42 %. Die Halbwertsbreite (FWHM) beträgt 73 nm.

**Beispiel 30**

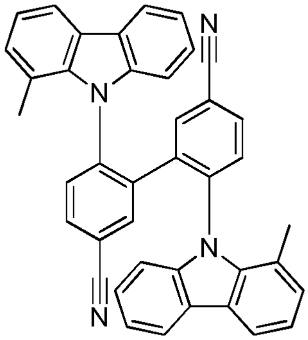

**30**

Die Filmemission von **30** (10 % in PMMA) wurde gemessen. Das Emissionsmaximum liegt bei 435 nm. Die Photolumineszenzquantenausbeute (PLQY) beträgt 41 %. Die Halbwertsbreite (FWHM) beträgt 77 nm.

**Beispiel 31**

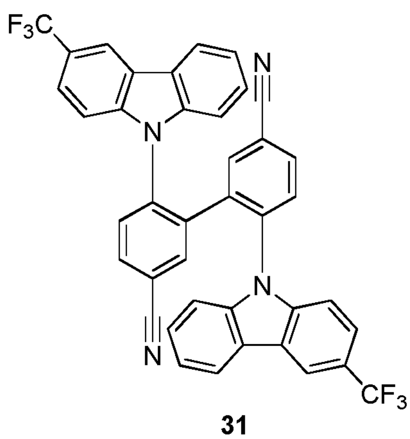

**31**

Die Filmemission von **31** (10 % in PMMA) wurde gemessen. Das Emissionsmaximum liegt bei 405 nm. Die

Photolumineszenzquantenausbeute (PLQY) beträgt 33 %. Die Halbwertsbreite (FWHM) beträgt 52 nm.

**Beispiel 32**

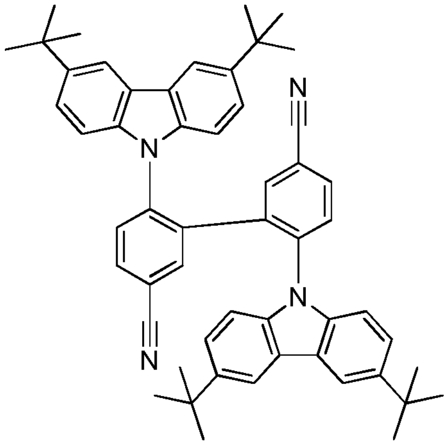

**32**

Die Filmemission von **32** (10 % in PMMA) wurde gemessen (Figur 8). Das Emissionsmaximum liegt bei 444 nm. Die Photolumineszenzquantenausbeute (PLQY) beträgt 68 %. Die Halbwertsbreite (FWHM) beträgt 59 nm.

Molekül **5** wurde in einem OLED-Bauteil ("Bauteil X3) mit folgendem Aufbau getestet (Anteil des erfindungsgemäßen Moleküls an der Emissionsschicht ist in Massenprozent angegeben):

| **Schicht** | **Dicke** | |
|---|---|---|
| **9** | 100 nm | Al |
| **8** | 2 nm | Liq |
| **7** | 30 nm | TPBi |
| **6** | 10 nm | DPEPO |
| **5** | 20 nm | **14** (40%):DPEPO |
| **4** | 10 nm | CzSi |
| **3** | 20 nm | TCTA |
| **2** | 70 nm | NPB |
| **1** | 120 nm | ITO |

| | |
|---|---|
| Leistungseffizienz: | 15,6 lm/W |
| Stromeffizienz: | 20,6 cd/A |
| CIE: | CIEx: 0,149<br>CIEy: 0,149<br>at 14 V |
| Externe Quantenausbeute (EQE): | 16,1% |

Weitere Beispiele organischer Moleküle mit einer erfindungsgemäßen Struktur:

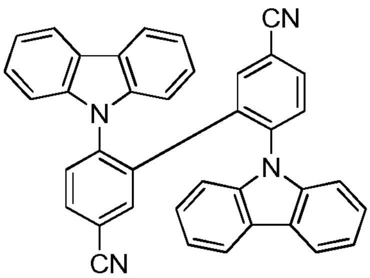

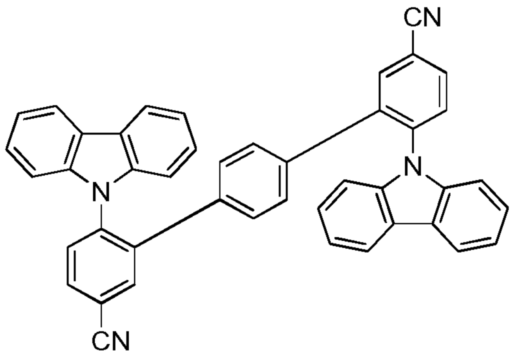

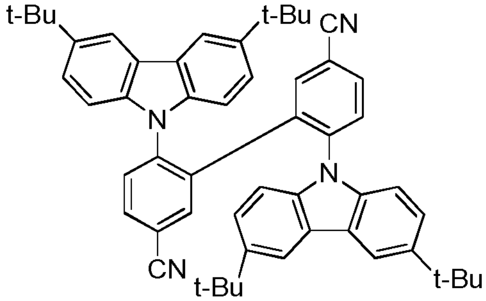

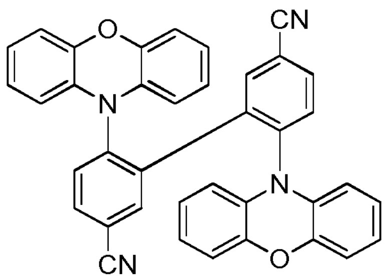

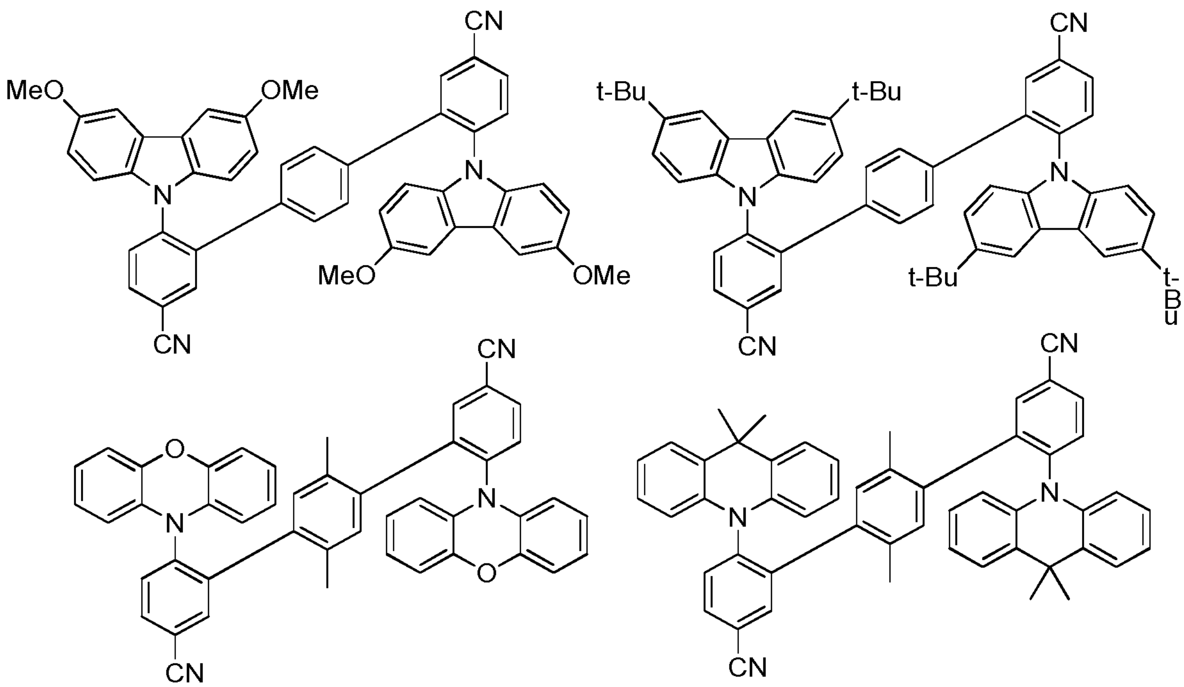

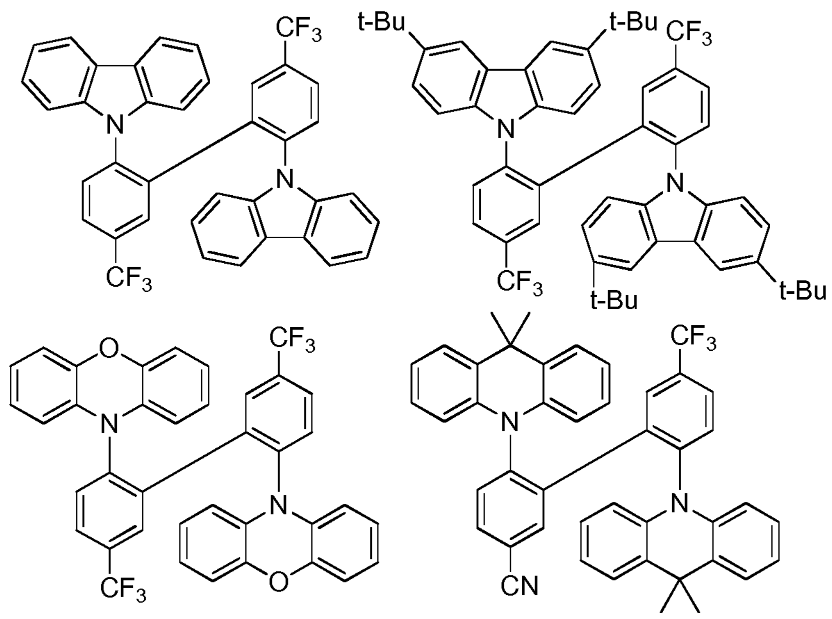

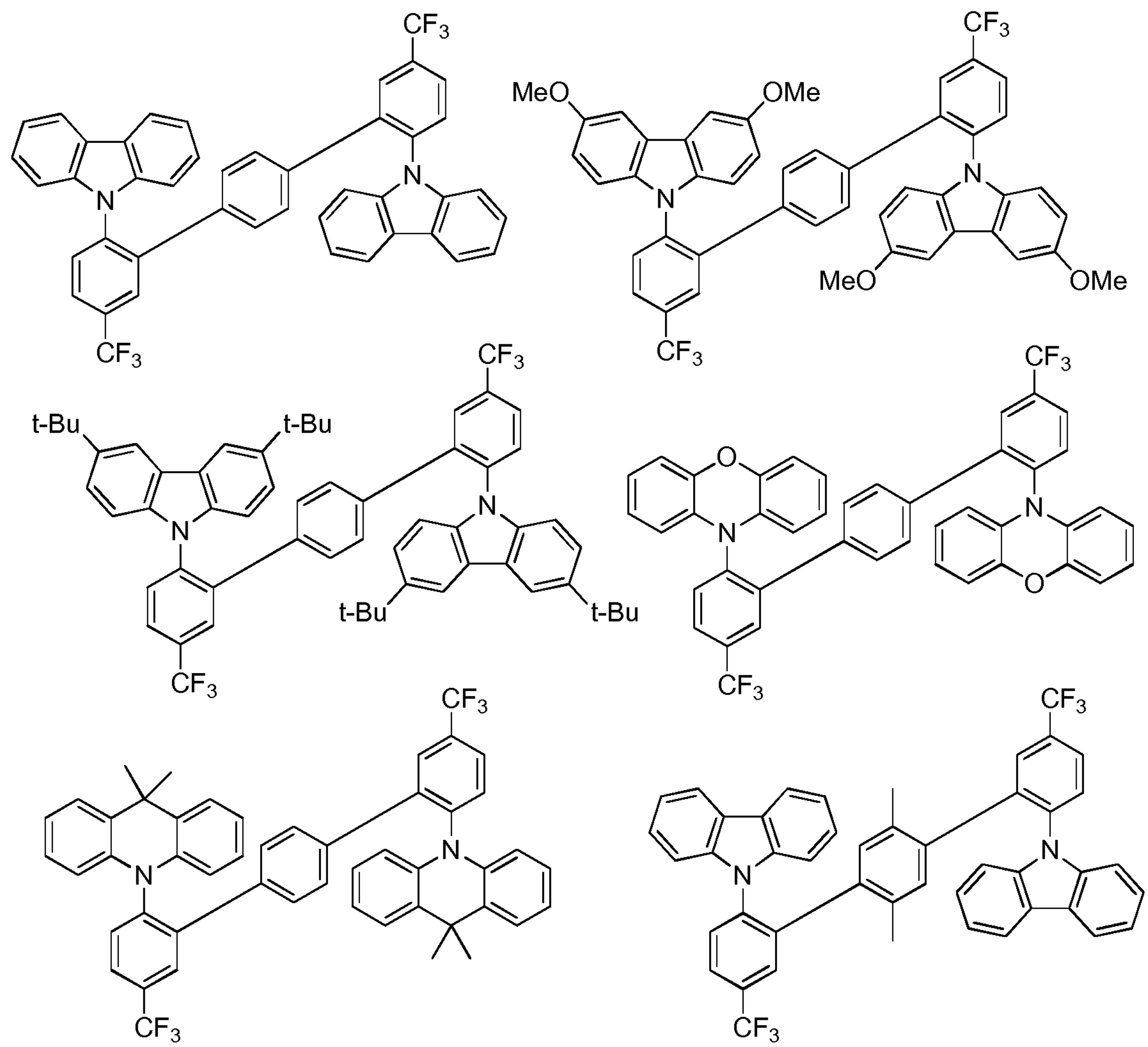

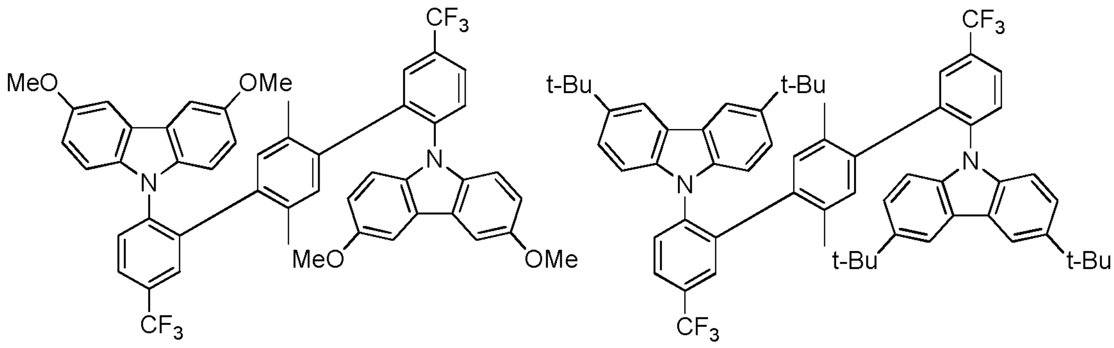

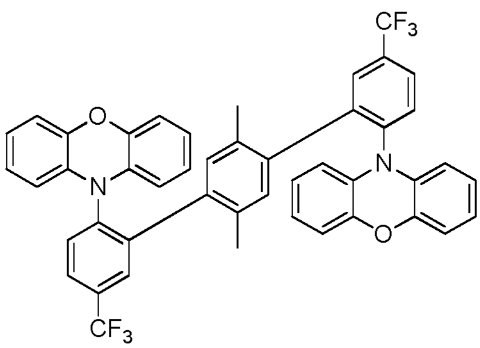

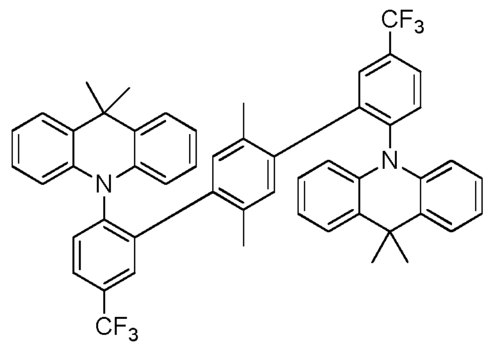

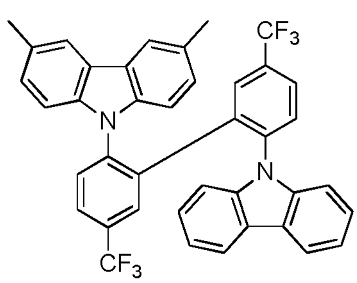

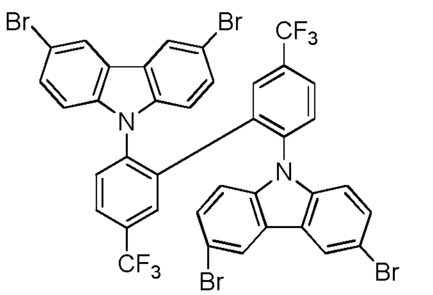

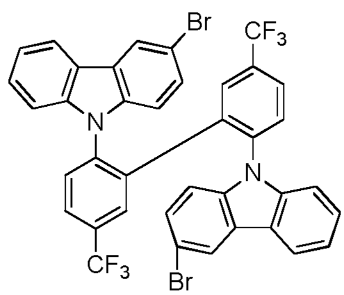

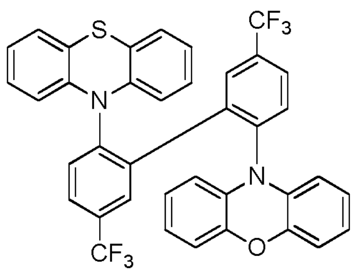

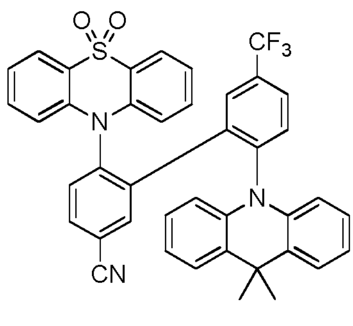

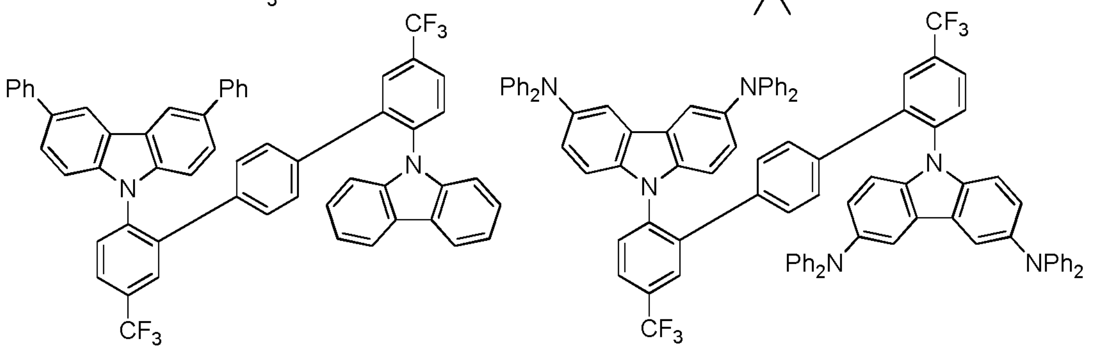

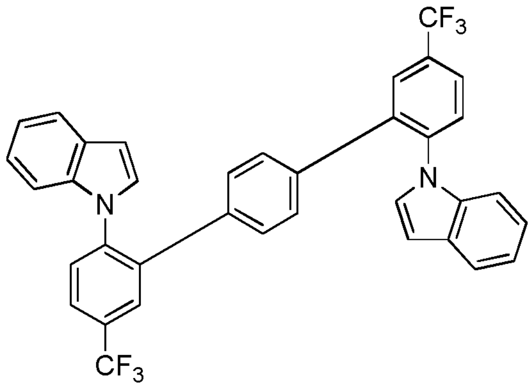

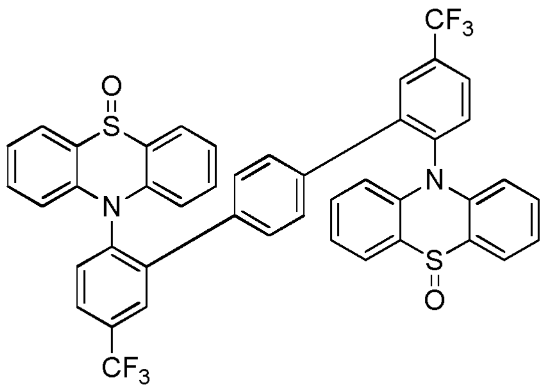

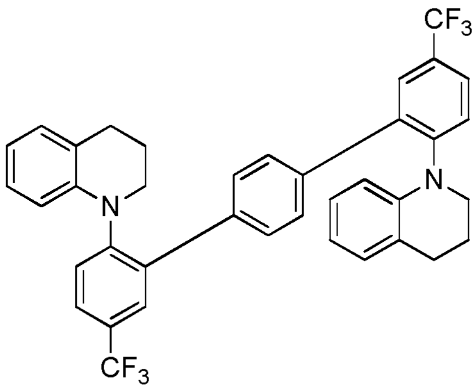

## Figuren

Figur 1: Filmemission von **1** (10 % in PMMA).
Figur 2: Filmemission von **3** (10 % in PMMA).
Figur 4: Filmemission von **4** (10 % in PMMA).
Figur 5: Filmemission von **5** (10 % in PMMA).
Figur 6: Filmemission von **6** (10 % in PMMA).
Figur 7: Filmemission von **7** (10 % in PMMA).
Figur 8: Filmemission von **32** (10 % in PMMA).
Figur 9: Filmemission von **9** (10 % in PMMA).
Figur 10: Filmemission von **10** (10 % in PMMA).
Figur 11: Filmemission von **11** (10 % in PMMA).
Figur 12: Stromdichte und Leuchtdichte des OLED-Bauteils X3.
Figur 13: Leistungseffizienz des OLED-Bauteils X3.
Figur 14: Stromeffizienz des OLED-Bauteils X3.
Figur 15: Externe Quanteneffizienz des OLED-Bauteils X3.
Figur 16: Elektrolumineszenzspektrum des OLED-Bauteils X3, betrieben bei 14 V.

## Patentansprüche

**1.** Organisches Molekül, aufweisend genau zwei Einheiten gemäß Formel I, die über eine Einfachbindung oder eine Brücke Y miteinander verknüpft sind,

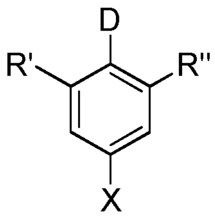

Formel I

mit

Y = eine divalente chemische Gruppe;
X = bei jedem Auftreten unabhängig voneinander ausgewählt aus der Gruppe bestehend aus CN und $CF_3$;
D = chemische Einheit aufweisend eine Struktur der Formel I-1:

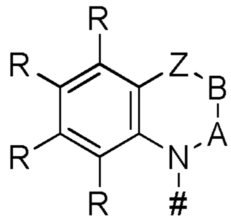

Formel I-1

wobei

# = Anknüpfungspunkt der Einheit gemäß Formel I-1 an die Struktur gemäß Formel I;
A und B = unabhängig voneinander ausgewählt aus der Gruppe bestehend aus $CRR^1$, CR, NR, N, wobei zwischen A und B eine Einfach- oder Doppelbindung vorliegt und zwischen B und Z eine Einfach- oder Doppelbindung vorliegt;
Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, $-CRR^1$, $-C=CRR^1$, -C=NR, -NR-, -O-, $-SiRR^1-$, -S-, -S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ist;
wobei jedes R und $R^1$ bei jedem Auftreten gleich oder verschieden ist H, Deuterium, Azid ($N_3^-$ ), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination dieser Systeme, oder eine quervernetzbare Einheit QE ist, die durch säurekatalytische, thermische oder UV-Quervernetzungsverfahren in Anwesenheit oder Abwesenheit eines Photoinitiators oder durch Mikrowellenstrahlung quervernetzt werden kann; wobei optional zwei oder mehrere dieser Substituenten R und $R^1$ miteinander ein mono- oder polycyclisches, aliphatisches, aromatisches und/oder benzoannelliertes Ringsystem bilden;
$R^2$ bei jedem Auftreten gleich oder verschieden ist H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; wobei optional zwei oder mehrere dieser Substituenten $R^2$ miteinander ein mono- oder polycyclisches, aliphatisches, aromatisches und/oder benzoannelliertes Ringsystem bilden;
$R^3$ bei jedem Auftreten gleich oder verschieden ist H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; wobei optional zwei oder mehrere Substituenten $R^3$ miteinander ein mono- oder polycyclisches, aliphatisches Ringsystem bilden;
R' = Anknüpfungsposition für die zweite Einheit der Formel I oder ausgewählt aus der Gruppe bestehend aus Y, H, $N(R^4)_2$, $OR^4$, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
R" = Anknüpfungsposition für die zweite Einheit der Formel I oder ausgewählt aus der Gruppe bestehend aus Y, $N(R^4)_2$, $OR^4$, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
mit der Maßgabe, dass wenn R' gleich Y ist, R" ungleich Y ist und wenn R" gleich Y ist, R' ungleich Y ist;
und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
$R^4$ bei jedem Auftreten gleich oder verschieden ist H, Deuterium, $N(R^5)_2$, $Si(R^5)_3$, eine lineare Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann, oder eine Kombination dieser Systeme; wobei optional zwei oder mehrere dieser Substituenten $R^5$ auch miteinander ein mono- oder polycyclisches, aliphatisches, aromatisches und/oder benzoannelliertes Ringsystem bilden; $R^5$ bei jedem Auftreten gleich oder verschieden ist H, Deuterium, oder ein aliphatischer, aromatischer und/oder heteroaromatischer Kohlenwasserstoffrest mit 1 bis 20 C-Atomen;
wobei optional zwei oder mehrere Substituenten $R^5$ auch miteinander ein mono- oder polycyclisches, aliphatisches Ringsystem bilden.

**2.** Organisches Molekül nach Anspruch 1, wobei
R' = Anknüpfungsposition für die zweite Einheit der Formel I oder ausgewählt aus der Gruppe bestehend aus Y, H, $N(R^4)_2$, $OR^4$, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
und R" = Anknüpfungsposition für die zweite Einheit der Formel I oder ausgewählt aus der Gruppe bestehend aus Y, $N(R^4)_2$, $OR^4$, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen oder heteroaromatischen Ringsystem mit 5 bis 60 aromatischen Ringatomen, das jeweils durch einen oder mehrere Reste $R^4$ substituiert sein kann;
wobei das heteroaromatische Ringsystem kein N-Heteroarom at ist;

wobei wenn R' gleich Y ist, R" ungleich Y ist und wenn R" gleich Y ist, R' ungleich Y ist;
und wobei wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
wobei Y und $R^4$ wie in Anspruch 1 definiert sind.

**3.** Organisches Molekül nach Anspruch 1 oder 2, wobei
R' = Anknüpfungsposition für die zweite Einheit der Formel I oder ausgewählt aus der Gruppe bestehend aus Y, H, $N(R^4)_2$, $OR^4$, Thiophen, das mit einem oder mehreren Resten $R^4$ substituiert sein kann, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen Ringsystem mit 5 bis 60 aromatischen Ringatomen, das durch einen oder mehrere Reste $R^4$ substituiert sein kann;
und R" = Anknüpfungsposition für die zweite Einheit der Formel I oder ausgewählt aus der Gruppe bestehend aus Y, $N(R^4)_2$, $OR^4$, Thiophen, das mit einer oder mehreren Resten$R^4$ substituiert sein kann, einer linearen Alkyl- oder Alkoxygruppe mit 1 bis 40 C-Atomen oder einer verzweigten oder cyclischen Alkyl- oder Alkoxygruppe mit 3 bis 40 C-Atomen, die jeweils mit einem oder mehreren Resten $R^4$ substituiert sein kann, und einem aromatischen Ringsystem mit 5 bis 60 aromatischen Ringatomen, das durch einen oder mehrere Reste $R^4$ substituiert sein kann;
wobei, wenn R' gleich Y ist, R" ungleich Y ist und wenn R" gleich Y ist, R' ungleich Y ist; und wobei, wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;
wobei Y und $R^4$ wie in Anspruch 1 definiert sind.

**4.** Organische Molekül nach Anspruch 1, wobei
R' gleich H ist und R" gleich dem Anknüpfungspunkt für die zweite Einheit der Formel I oder gleich Y ist;
wobei Y wie in Anspruch 1 definiert ist.

**5.** Organisches Molekül nach Anspruch 1 bis 4, wobei die Donorgruppe mit elektronenschiebenden Eigenschaften der Formel I-1 eine Struktur der Formel II aufweist:

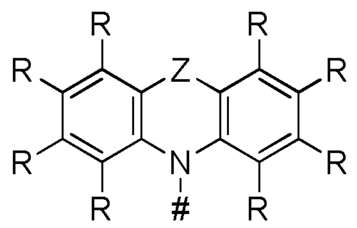

Formel II

wobei für #, Z und R die in Anspruch 1 genannten Definitionen gelten.

**6.** Organisches Molekül nach Anspruch 1 bis 5, wobei die Donorgruppe mit elektronenschiebenden Eigenschaften der Formel I-1 eine Struktur der Formel III aufweist:

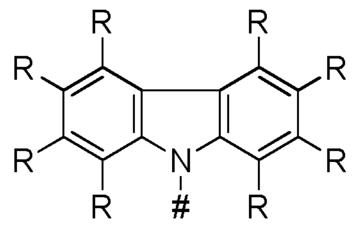

Formel III

wobei für # und R die in Anspruch 1 genannten Definitionen gelten.

**7.** Organisches Molekül nach Anspruch 1,5, 6 oder 7, wobei das Molekül eine Struktur der Formel XII aufweist:

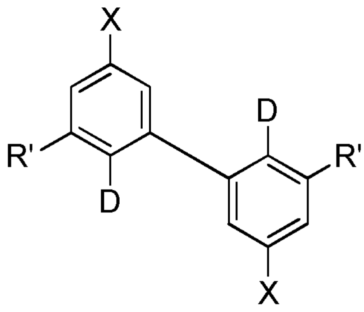

Formel XII

mit n = 0 oder 1;
und wobei für D, R’ und X die in Anspruch 1 genannten Definitionen gelten,
wobei insbesondere R’ gleich H ist.

**8.** Organisches Molekül nach Anspruch 1 bis 7, wobei die akzeptierende Einheit X der Formel I gleich CN ist oder wobei die akzeptierende Einheit X der Formel I gleich $CF_3$ ist.

**9.** Organisches Molekül nach Anspruch 1 bis 6 oder 8, wobei die Brücke Y ausgewählt ist aus den Strukturen der Formeln IV bis X

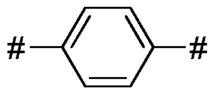

IV

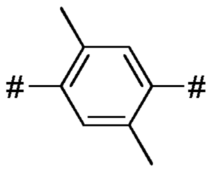

V

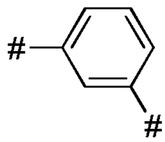

VI

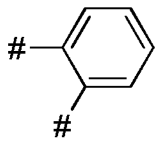

VII

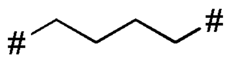

VIII

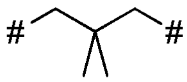

IX

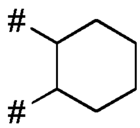

X

mit
# = Anknüpfungspunkt der Brücke Y an die Struktur gemäß Formel I.

**10.** Verfahren zur Herstellung eines organischen Moleküls nach Anspruch 1 bis 9.

**11.** Verwendung eines organischen Moleküls nach Anspruch 1 bis 9 als lumineszierender Emitter oder als Absorber und/oder als Hostmaterial und/oder als Elektronentransportmaterial und/oder als Lochinjektionsmaterial und/oder als Lochblockiermaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**12.** Verwendung nach Anspruch 11, wobei der Anteil des organischen Moleküls an der Emissionsschicht in einer organischen optoelektronischen Vorrichtung, insbesondere in OLEDs, 1 % bis 99 %, insbesondere 5 % bis 80 % beträgt.

**13.** Organische optoelektronische Vorrichtung, aufweisend ein organisches Molekül nach Anspruch 1 bis 9, insbesondere ausgeformt als eine Vorrichtung ausgewählt aus der Gruppe bestehend aus organischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**14.** Organische optoelektronische Vorrichtung nach Anspruch 13, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organische Molekül nach Anspruch 1 bis 9 aufweist.

**15.** Verfahren zur Herstellung eines optoelektronischen Bauelements, wobei ein organisches Molekül nach Anspruch 1 bis 9 verwendet wird, insbesondere umfassend die Verarbeitung des organischen Moleküls mittels eines Vakuumverdampfungsverfahrens oder aus einer Lösung.

**Claims**

**1.** Organic molecule having precisely two units of formula I linked to one another via a single bond or a bridge Y,

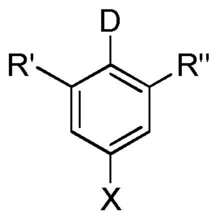

Formula I

wherein

Y = a divalent chemical group;
X = at each occurrence independently of one another selected from the group consisting of CN and $CF_3$;
D = a chemical unit having a structure of the formula I-1:

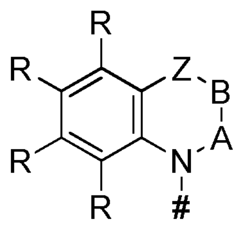

Formula I-1

wherein

# = attachment point of the unit of formula I-1 to the structure of formula I;
A and B = independently of one another selected from the group consisting of $CRR^1$, CR, NR, N, there being a single or double bond between A and B and a single or double bond between B and Z;
Z = a direct bond or a divalent organic bridge which is a substituted or unsubstituted C1-C9-alkylene, C2-C8-alkenylene, C2-C8-alkynylene or arylene group or a combination thereof, $-CRR^1$, $-C=CRR^1$, -C=NR, -NR-, -O-, $-SiRR^1-$, -S-, -S(O)-, $-S(O)_2-$, O-interrupted substituted or unsubstituted C1-C9-alkylene, C2-C8-alkenylene, C2-C8-alkynylene or arylene group, phenyl units or substituted phenyl units;

wherein each R and $R^1$, identically or differently at each occurrence, is H, deuterium, azide ($N_3^-$ ), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a linear alkyl, alkoxy or thioalkoxy group having 1 to 40 C atoms or a linear alkenyl or alkynyl group having 2 to 40 C atoms or a branched or cyclic alkyl, alkenyl, alkynyl, alkoxy or thioalkoxy group having 3 to 40 C atoms, which in each case may be substituted by one or more radicals $R^2$, it being possible for one or more non-adjacent $CH_2$ groups to be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$, and it being possible for one or more H atoms to be replaced by deuterium, F, Cl, Br, I, CN, $CF_3$ or $NO_2$, or is an aromatic or heteroaromatic ring system having 5 to 60 aromatic ring atoms, it being possible for this ring system to be substituted in each case by one or more radicals $R^2$, or is an aryloxy or heteroaryloxy group having 5 to 60 aromatic ring atoms, it being possible for this group to be substituted by one or more radicals $R^2$, or is a diarylamino group, diheteroarylamino group or arylheteroarylamino group having 10 to 40 aromatic ring atoms, it being possible for this group to be substituted by one or more radicals $R^2$, or is a combination of these systems, or is a crosslinkable unit QE which may be crosslinked by acid-catalytic, thermal or UV crosslinking methods in the presence or absence of a photoinitiator or by microwave radiation; optionally two or more of these substituents R and $R^1$ may form with one another a mono- or polycyclic, aliphatic, aromatic and/or benzo-fused ring system;

$R^2$, identically or differently at each occurrence, is H, deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a linear alkyl, alkoxy or thioalkoxy group having 1 to 40 C atoms or a linear alkenyl or alkynyl group having 2 to 40 C atoms or a branched or cyclic alkyl, alkenyl, alkynyl, alkoxy or thioalkoxy group having 3 to 40 C atoms, which may be substituted in each case by one or more radicals $R^3$, it being possible for one or more non-adjacent $CH_2$ groups to be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$, and it being possible for one or more H atoms to be replaced by deuterium, F, Cl, Br, I, CN, $CF_3$ or $NO_2$, or is an aromatic or heteroaromatic ring system having 5 to 60 aromatic ring atoms, it being possible for this system to be substituted in each case by one or more radicals $R^3$, or is an aryloxy or heteroaryloxy group having 5 to 60 aromatic ring atoms, it being possible for this group to be substituted by one or more radicals $R^3$, or is a diarylamino group, diheteroarylamino group or arylheteroarylamino group having 10 to 40 aromatic ring atoms, it being possible for this group to be substituted by one or more radicals $R^3$, or is a combination of these systems; wherein optionally two or more of these substituents $R^2$ form with one another a mono- or polycyclic, aliphatic, aromatic and/or benzo-fused ring system;

$R^3$, identically or differently at each occurrence, is H, deuterium, F, $CF_3$ or an aliphatic, aromatic and/or heteroaromatic hydrocarbon radical having 1 to 20 C atoms, in which also one or more H atoms may be replaced by F or $CF_3$; wherein optionally two or more substituents $R^3$ form with one another a mono- or polycyclic, aliphatic ring system;

R' = attachment position for the second unit of the formula I or selected from the group consisting of Y, H, $N(R^4)_2$, $OR^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic or heteroaromatic ring system having 5 to 60 aromatic ring atoms, it being possible for this system to be substituted in each case by one or more radicals $R^4$;

R" = attachment position for the second unit of the formula I or selected from the group consisting of Y, $N(R^4)_2$, $OR^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic or heteroaromatic ring system having 5 to 60 aromatic ring atoms, it being possible for this system to be substituted in each case by one or more radicals $R^4$;

with the proviso that if R' is Y, R" is not Y, and, if R" is Y, R' is not Y;

and with the proviso that if R' is the attachment position for the second unit of the formula I, R" is not the attachment position for the second unit of the formula I, and, if R" is the attachment position for the second unit of the formula I, R' is not the attachment position for the second unit of the formula I;

$R^4$, identically or differently at each occurrence, is H, deuterium, $N(R^5)_2$, $Si(R^5)_3$, a linear alkyl, alkoxy or thioalkoxy group having 1 to 40 C atoms or a branched or cyclic alkyl, alkoxy or thioalkoxy group having 3 to 40 C atoms, it being possible for this group to be substituted in each case by one or more radicals $R^5$, or is an aromatic or heteroaromatic ring system having 5 to 60 aromatic ring atoms, it being possible for this system to be substituted in each case by one or more radicals $R^5$, or is an aryloxy or heteroaryloxy

group having 5 to 60 aromatic ring atoms, it being possible for this group to be substituted by one or more radicals $R^5$, or is a diarylamino group, diheteroarylamino group or arylheteroarylamino group having 10 to 40 aromatic ring atoms, it being possible for this group to be substituted by one or more radicals $R^5$, or is a combination of these systems; wherein optionally two or more of these substituents $R^5$ may also form with one another a mono- or polycyclic, aliphatic, aromatic and/or benzo-fused ring system;
$R^5$, identically or differently at each occurrence, is H, deuterium or an aliphatic, aromatic and/or heteroaromatic hydrocarbon radical having 1 to 20 C atoms; wherein optionally two or more substituents $R^5$ may also form with one another a mono- or polycyclic, aliphatic ring system.

**2.** Organic molecule according to Claim 1, wherein
R' = attachment position for the second unit of the formula I or selected from the group consisting of Y, H, $N(R^4)_2$, $OR^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic or heteroaromatic ring system having 5 to 60 aromatic ring atoms, it being possible for this system to be substituted in each case by one or more radicals $R^4$;
and R" = attachment position for the second unit of the formula I or selected from the group consisting of Y, $N(R^4)_2$, $OR^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic or heteroaromatic ring system having 5 to 60 aromatic ring atoms, it being possible for this system to be substituted in each case by one or more radicals $R^4$;
wherein the heteroaromatic ring system is not an N-heteroaromatic;
wherein, if R' is Y, R" is not Y, and, if R" is Y, R' is not Y;
and wherein, if R' is the attachment position for the second unit of the formula I, R" is not the attachment position for the second unit of the formula I, and, if R" is the attachment position for the second unit of the formula I, R' is not the attachment position for the second unit of the formula I;
wherein Y and $R^4$ are as defined in Claim 1.

**3.** Organic molecule according to Claim 1 or 2, wherein
R' = attachment position for the second unit of the formula I or selected from the group consisting of Y, H, $N(R^4)_2$, $OR^4$, thiophene which may be substituted by one or more radicals $R^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic ring system having 5 to 60 aromatic ring atoms, it being possible for this system to be substituted by one or more radicals $R^4$;
and R" = attachment position for the second unit of the formula I or selected from the group consisting of Y, $N(R^4)_2$, $OR^4$, thiophene which may be substituted by one or more radicals $R^4$, a linear alkyl or alkoxy group having 1 to 40 C atoms or a branched or cyclic alkyl or alkoxy group having 3 to 40 C atoms, it being possible for this group to be substituted in each case by one or more radicals $R^4$, and an aromatic ring system having 5 to 60 aromatic ring atoms, it being possible for this system to be substituted by one or more radicals $R^4$; wherein, if R' is Y, R" is not Y, and, if R" is Y, R' is not Y;
and wherein, if R' is the attachment position for the second unit of the formula I, R" is not the attachment position for the second unit of the formula I, and, if R" is the attachment position for the second unit of the formula I, R' is not the attachment position for the second unit of the formula I;
wherein Y and $R^4$ are as defined in Claim 1.

**4.** Organic molecule according to Claim 1, wherein
R' is H and R" is the attachment point for the second unit of the formula I or is Y; wherein Y is as defined in Claim 1.

**5.** Organic molecule according to Claim 1 to 4, wherein the donor group having electron-donating properties of the formula I-1 has a structure of the formula II:

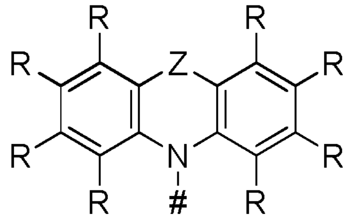

Formula II

wherein #, Z and R are defined as stated in Claim 1.

6. Organic molecule according to Claim 1 to 5, wherein the donor group having electronic-donating properties of the formula I-1 has a structure of the formula III:

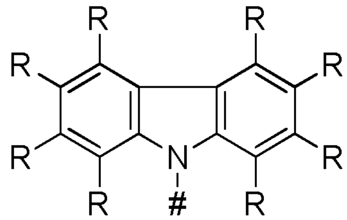

Formula III

wherein # and R are defined as stated in Claim 1.

7. Organic molecule according to Claim 1, 5, 6 or 7, wherein the molecule has a structure of the formula XII:

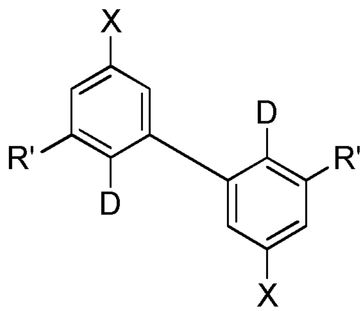

Formula XII

wherein n = 0 or 1;
and wherein D, R' and X are defined as stated in Claim 1, in particular wherein R' is H.

8. Organic molecule according to Claim 1 to 7, wherein the accepting unit X of the formula I is CN or wherein the accepting unit X of the formula I is $CF_3$.

9. Organic molecule according to Claim 1 to 6 or 8, wherein the bridge Y is selected from the structures of the formulae IV to X

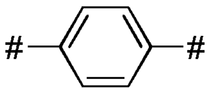

IV

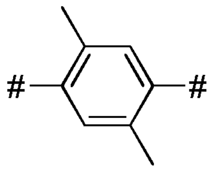

V

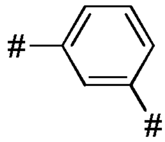

VI

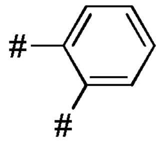

VII

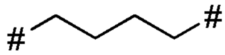

VIII

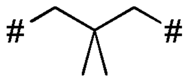

IX

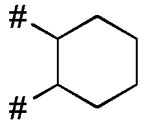

X

wherein # = attachment point of the bridge Y to the structure of formula I.

**10.** Process for preparing an organic molecule according to Claim 1 to 9.

**11.** Use of an organic molecule according to Claim 1 to 9 as luminescent emitter or as an absorber and/or as a host material and/or as an electron transport material and/or as a hole injection material and/or as a hole blocking material in an organic optoelectronic device, in particular wherein the organic optoelectronic device is selected from the group consisting of:

- organic light-emitting diodes (OLEDs),
- light-emitting electrochemical cells,
- OLED sensors, especially in gas and vapour sensors not hermetically externally shielded,
- organic diodes,
- organic solar cells,
- organic transistors,
- organic field-effect transistors,
- organic lasers and
- down-conversion elements.

**12.** Use according to Claim 11, wherein the fraction of the organic molecule in the emission layer in an organic optoelectronic device, more particularly in OLEDs, is 1 % to 99 %, more particularly 5 % to 80 %.

**13.** Organic optoelectronic device comprising an organic molecule according to Claim 1 to 9, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

**14.** Organic optoelectronic device according to Claim 13, comprising

- a substrate,
- an anode and
- a cathode, the anode or the cathode being applied to the substrate, and
- at least one light-emitting layer which is disposed between anode and cathode and which comprises the organic molecule according to Claim 1 to 9.

**15.** Process for producing an optoelectronic component, wherein an organic molecule according to Claim 1 to 9 is used, in particular comprising the processing of the organic molecule by means of a vacuum vaporization process or from a solution.

**Revendications**

1. Molécule organique présentant exactement deux unités selon la formule I, qui sont liées l'une à l'autre via une simple liaison ou un pont Y

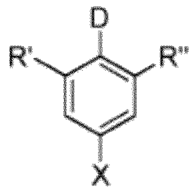

Formule I

où

Y = un groupe chimique bivalent ;
X = choisi en chaque occurrence, indépendamment, dans le groupe constitué par CN et $CF_3$ ;
D = une unité chimique présentant une structure de formule I-1 :

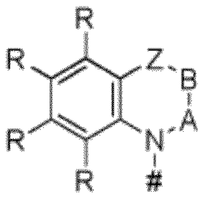

Formule I-1

dans laquelle

# = le point de liaison de l'unité selon la formule I-1 à la structure selon la formule I ; A et B = choisis, indépendamment l'un de l'autre, dans le groupe constitué par $CRR^1$, CR, NR, N, une simple liaison ou une double liaison étant située entre A et B et une simple liaison ou une double liaison étant située entre B et Z ; Z = une liaison directe ou un pont organique bivalent, qui est un groupe $C_1$-$C_9$-alkylène, $C_2$-$C_8$-alcénylène, $C_2$-$C_8$-alcynylène ou arylène substitué ou non substitué ou une combinaison de ceux-ci, $-CRR^1$, $-C=CRR^1$, -C=NR, -NR-, -O-, $-SiRR^1$-, -S-, -S(O)-, $-S(O)_2$- ; un groupe $C_1$-$C_9$-alkylène, $C_2$-$C_8$-alcénylène, $C_2$-$C_8$-alcynylène ou arylène substitué ou non substitué, interrompu par O ; des unités phényle ou des unités phényle substituées ; chaque R et $R^1$, en chaque occurrence, de manière identique ou différente, représentant H, deutérium, azide ($N_3$-), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy linéaire comprenant 1 à 40 atomes de carbone ou un groupe alcényle ou alcynyle linéaire comprenant 2 à 40 atomes de carbone ou un groupe alkyle, alcényle, alcynyle, alcoxy ou thioalcoxy ramifié ou cyclique comprenant 3 à 40 atomes de carbone, qui peut à chaque fois être substitué par un ou plusieurs radicaux $R^2$, un ou plusieurs groupes $CH_2$ non adjacents pouvant être remplacés par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$, ou un système cyclique aromatique ou hétéroaromatique comprenant 5 à 60 atomes de cycle aromatique, qui peut à chaque fois être substitué par un ou plusieurs radicaux $R^2$, ou un groupe aryloxy ou hétéroaryloxy comprenant 5 à 60 atomes de cycle aromatique, qui peut être substitué par un ou plusieurs radicaux $R^2$, ou un groupe diarylamino, un groupe dihétéroarylamino ou un groupe arylhétéroarylamino comprenant 10 à 40 atomes de cycle aromatique, qui peut être substitué par un ou plusieurs radicaux $R^2$, ou une combinaison de ces systèmes, ou une unité réticulable QE, qui peut être réticulée par des procédés de réticulation catalytiques acides, thermiques ou UV en présence ou en l'absence d'un photo-initiateur ou par un rayonnement de micro-ondes ; deux de ces substituants R et $R^1$ ou plus pouvant éventuellement former l'un avec l'autre un système

cyclique monocyclique ou polycyclique, aliphatique, aromatique ou et/ou benzocondensé;
$R^2$ en chaque occurrence, de manière identique ou différente, représentant H, deutérium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy linéaire comprenant 1 à 40 atomes de carbone ou un groupe alcényle ou alcynyle linéaire comprenant 2 à 40 atomes de carbone ou un groupe alkyle, alcényle, alcynyle, alcoxy ou thioalcoxy ramifié ou cyclique comprenant 3 à 40 atomes de carbone, qui peut à chaque fois être substitué par un ou plusieurs radicaux $R^3$, un ou plusieurs groupes $CH_2$ non adjacents pouvant être remplacés par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et un ou plusieurs atomes de H pouvant être remplacés par deutérium, F, Cl, Br, I, CN, $CF_3$ ou $NO_2$ ou un système cyclique aromatique ou hétéroaromatique comprenant 5 à 60 atomes de cycle aromatique, qui peut à chaque fois être substitué par un ou plusieurs radicaux $R^3$, ou un groupe aryloxy ou hétéroaryloxy comprenant 5 à 60 atomes de cycle aromatique, qui peut être substitué par un ou plusieurs radicaux $R^3$, ou un groupe diarylamino, un groupe dihétéroarylamino ou un groupe arylhétéroarylamino comprenant 10 à 40 atomes de cycle aromatique, qui peut être substitué par un ou plusieurs radicaux $R^3$, ou une combinaison de ces systèmes ; deux de ces substituants $R^2$ ou plus pouvant éventuellement former l'un avec l'autre un système cyclique monocyclique ou polycyclique, aliphatique, aromatique et/ou benzocondensé ;
$R^3$ en chaque occurrence, de manière identique ou différente, représentant H, deutérium, F, $CF_3$ ou un radical hydrocarboné aliphatique, aromatique et/ou hétéroaromatique comprenant 1 à 20 atomes de carbone, dans lequel un ou plusieurs atomes H peuvent également être remplacés par F ou $CF_3$ ; deux substituants $R^3$ ou plus formant éventuellement l'un avec l'autre un système cyclique monocyclique ou polycyclique, aliphatique ;
R' = position de liaison pour la deuxième unité de formule I ou choisi dans le groupe constitué par Y, H, $N(R^4)_2$, $OR^4$, un groupe alkyle ou alcoxy linéaire comprenant 1 à 40 atomes de carbone ou un groupe alkyle ou alcoxy ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
R" = position de liaison pour la deuxième unité de formule I ou choisi dans le groupe constitué par Y, $N(R^4)_2$, $OR^4$, un groupe alkyle ou alcoxy linéaire comprenant 1 à 40 atomes de carbone ou un groupe alkyle ou alcoxy ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
à condition que, lorsque R' représente Y, R" soit différent de Y et, lorsque R" représente Y, R' soit différent de Y ;
et à condition que, lorsque R' représente la position de liaison pour la deuxième unité de formule I, R" soit différent de la position de liaison pour la deuxième unité de formule I et, lorsque R" représente la position de liaison pour la deuxième unité de formule I, R' soit différent de la position de liaison pour la deuxième unité de formule I ;
$R^4$ en chaque occurrence, de manière identique ou différente, représentant H, deutérium, $N(R^5)_2$, $Si(R^5)_3$, un groupe alkyle, alcoxy ou thioalcoxy linéaire comprenant 1 à 40 atomes de carbone ou un groupe alkyle, alcoxy ou thioalcoxy ramifié ou cyclique comprenant 3 à 40 atomes de carbone, qui peut à chaque fois être substitué par un ou plusieurs radicaux $R^5$, ou un système cyclique aromatique ou hétéroaromatique comprenant 5 à 60 atomes de cycle aromatique, qui peut à chaque fois être substitué par un ou plusieurs radicaux $R^5$, ou un groupe aryloxy ou hétéroaryloxy comprenant 5 à 60 atomes de cycle aromatique, qui peut à chaque fois être substitué par un ou plusieurs radicaux $R^5$, ou un groupe diarylamino, un groupe dihétéroarylamino ou un groupe arylhétéroarylamino comprenant 10 à 40 atomes de cycle aromatique, qui peut être substitué par un ou plusieurs radicaux $R^5$, ou une combinaison de ces systèmes ; deux de ces substituants $R^5$ ou plus pouvant éventuellement aussi former l'un avec l'autre un système cyclique monocyclique ou polycyclique, aliphatique, aromatique et/ou benzocondensé;
$R^5$ en chaque occurrence, de manière identique ou différente, représentant H, deutérium, ou un radical hydrocarboné aliphatique, aromatique et/ou hétéroaromatique comprenant 1 à 20 atomes de carbone ; deux substituants $R^5$ ou plus formant éventuellement également l'un avec l'autre un système cyclique monocyclique ou polycyclique, aliphatique.

**2.** Molécule organique selon la revendication 1, où
R' = position de liaison pour la deuxième unité de formule I ou choisi dans le groupe constitué par Y, H, $N(R^4)_2$, $OR^4$, un groupe alkyle ou alcoxy linéaire comprenant 1 à 40 atomes de carbone ou un groupe alkyle ou alcoxy

ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
et R" = position de liaison pour la deuxième unité de formule I ou choisi dans le groupe constitué par Y, $N(R^4)_2$, $OR^4$, un groupe alkyle ou alcoxy linéaire comprenant 1 à 40 atomes de carbone ou un groupe alkyle ou alcoxy ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
le système cyclique hétéroaromatique n'étant pas un N-hétéroaromatique ;
où, lorsque R' représente Y, R" est différent de Y et, lorsque R" représente Y, R' est différent de Y;
et où, lorsque R' représente la position de liaison pour la deuxième unité de formule I, R" est différent de la position de liaison pour la deuxième unité de formule I et, lorsque R" représente la position de liaison pour la deuxième unité de formule I, R' est différent de la position de liaison pour la deuxième unité de formule I ;
Y et $R^4$ étant définis comme dans la revendication 1.

3. Molécule organique selon la revendication 1 ou 2, où
R' = position de liaison pour la deuxième unité de formule I ou choisi dans le groupe constitué par Y, H, $N(R^4)_2$, $OR_4$, thiophène, qui peut être substitué par un ou plusieurs radicaux $R^4$, un groupe alkyle ou alcoxy linéaire comprenant 1 à 40 atomes de carbone ou un groupe alkyle ou alcoxy ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
et R" = position de liaison pour la deuxième unité de formule I ou choisi dans le groupe constitué par Y, H, $N(R^4)_2$, $OR^4$, thiophène, qui peut être substitué par un ou plusieurs radicaux $R^4$, un groupe alkyle ou alcoxy linéaire comprenant 1 à 40 atomes de carbone ou un groupe alkyle ou alcoxy ramifié ou cyclique comprenant 3 à 40 atomes de carbone, qui peut à chaque fois être substitué par un ou plusieurs radicaux $R^4$, et un système cyclique aromatique ou hétéroaromatique comprenant 5 à 60 atomes de cycle aromatique, qui peut à chaque fois être substitué par un ou plusieurs radicaux $R^4$ ;
où, lorsque R' représente Y, R" est différent de Y et, lorsque R" représente Y, R' est différent de Y;
et où, lorsque R' représente la position de liaison pour la deuxième unité de formule I, R" est différent de la position de liaison pour la deuxième unité de formule I et, lorsque R" représente la position de liaison pour la deuxième unité de formule I, R' est différent de la position de liaison pour la deuxième unité de formule I ;
Y et $R^4$ étant définis comme dans la revendication 1.

4. Molécule organique selon la revendication 1, où
R' représente H et R" représente le point de liaison pour la deuxième unité de formule I ou Y ; Y étant défini comme dans la revendication 1.

5. Molécule organique selon les revendications 1 à 4, le groupe donneur présentant des propriétés de déplacement d'électrons de formule I-1 présentant une structure de formule II :

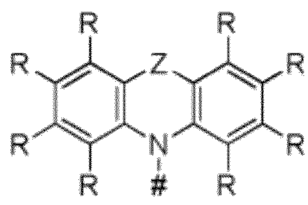

Formule II

les mêmes définitions que celles dans la revendication 1 étant d'application pour #, Z et R.

6. Molécule organique selon les revendications 1 à 5, le groupe donneur présentant des propriétés de déplacement d'électrons de formule I-1 présentant une structure de formule III :

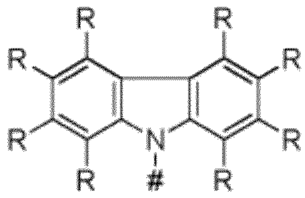

Formule III

les mêmes définitions que celles dans la revendication 1 étant d'application pour # et R.

**7.** Molécule organique selon les revendications 1, 5, 6 ou 7, la molécule présentant une structure de formule XII :

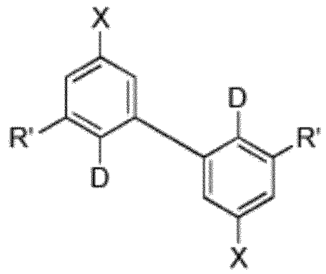

Formule XII

avec

n = 0 ou 1 ;
les mêmes définitions que celles dans la revendication 1 étant d'application pour D, R' et X, R' représentant en particulier H.

**8.** Molécule organique selon les revendications 1 à 7, l'unité accepteuse X de formule I représentant CN ou l'unité accepteuse X de formule I représentant $CF_3$.

**9.** Molécule organique selon les revendications 1 à 6 ou 8, le pont Y étant choisi parmi les structures des formules IV à X :

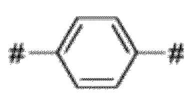 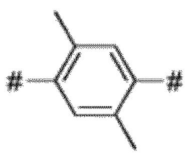 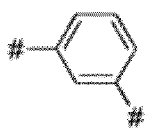 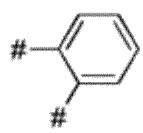

IV V VI VII

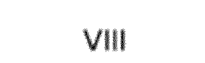 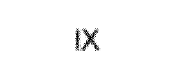 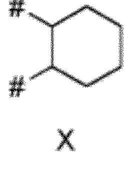

VIII IX X

où
# = point de liaison du pont Y à la structure selon la formule I.

**10.** Procédé pour la préparation d'une molécule organique selon les revendications 1 à 9.

**11.** Utilisation d'une molécule organique selon les revendications 1 à 9 comme émetteur électroluminescent ou comme

absorbant et/ou comme matériau hôte et/ou comme matériau de transport d'électrons et/ou comme matériau d'injection de trous et/ou comme matériau de blocage de trous dans un dispositif optoélectronique organique, le dispositif optoélectronique organique étant en particulier choisi dans le groupe constitué par :

• les diodes électroluminescentes organiques (DELO),
• les piles électrochimiques électroluminescentes,
• les capteurs à DELO, en particulier dans les capteurs de gaz et de vapeur, non hermétiquement fermés par rapport à l'extérieur,
• les diodes organiques.
• les piles solaires organiques,
• les transistors organiques.
• les transistors organiques à effet de champ,
• les lasers organiques et
• les éléments de conversion vers le bas.

**12.** Utilisation selon la revendication 11, la proportion de molécule organique dans la couche émettrice dans un dispositif optoélectronique organique, en particulier dans les DELO, étant de 1% à 99%, en particulier de 5% à 80%.

**13.** Dispositif optoélectronique organique, présentant une molécule organique selon les revendications 1 à 9, en particulier formé sous forme d'un dispositif choisi dans le groupe constitué par les diodes électroluminescentes organiques (DELO), une pile électrochimique électroluminescente, un capteur à DELO, en particulier dans les capteurs de gaz et de vapeur, non hermétiquement fermés par rapport à l'extérieur, les diodes organiques, les piles solaires organiques, un transistor organique, un transistor organique à effet de champ, un laser organique et un élément de conversion vers le bas.

**14.** Dispositif optoélectronique organique selon la revendication 13, présentant

- un substrat,
- une anode et
- une cathode, l'anode ou la cathode étant appliquées sur le substrat et
- au moins une couche électroluminescente, qui est disposée entre l'anode et la cathode et qui présente la molécule organique selon les revendications 1 à 9.

**15.** Procédé pour la fabrication d'un composant optoélectronique, une molécule organique selon les revendications 1 à 9 étant utilisée, comprenant en particulier la transformation de la molécule organique au moyen d'un procédé d'évaporation sous vide ou à partir d'une solution.

Figur 1

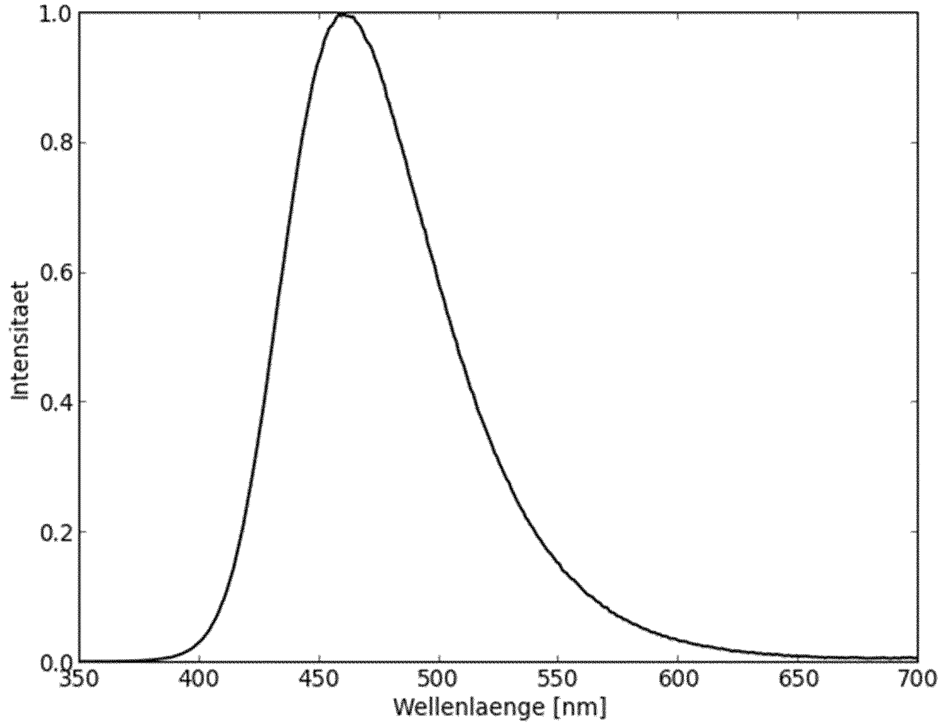


Figur 2

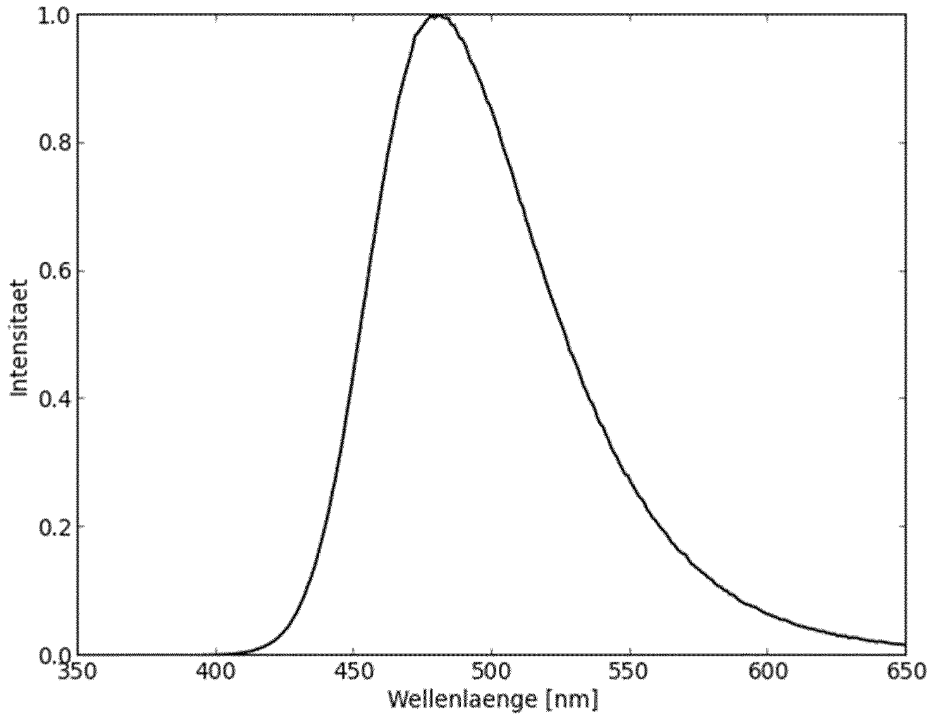

Figur 3

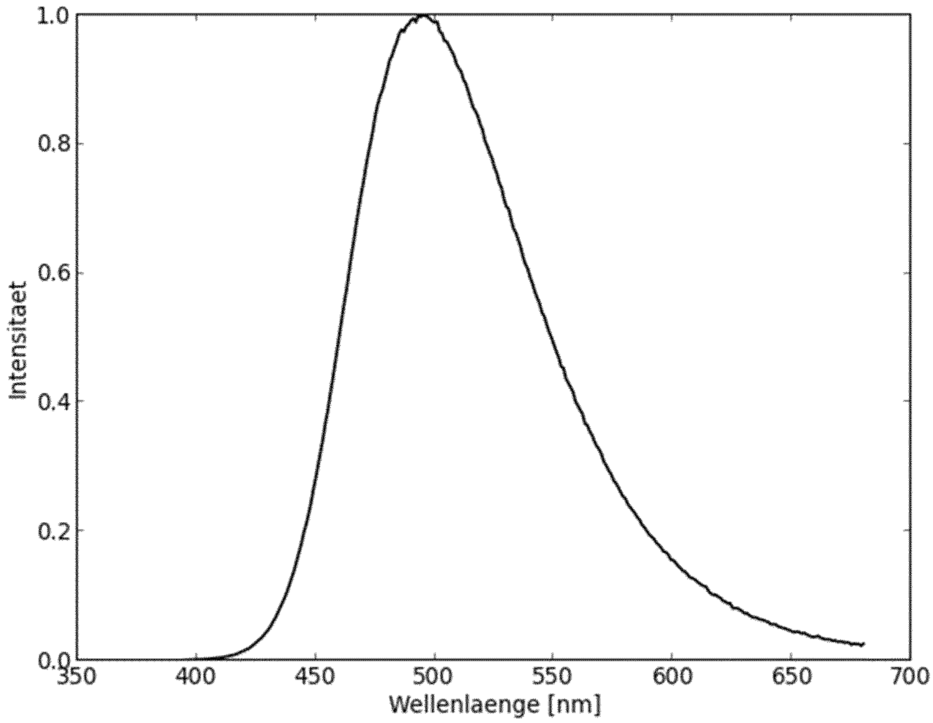


Figur 4

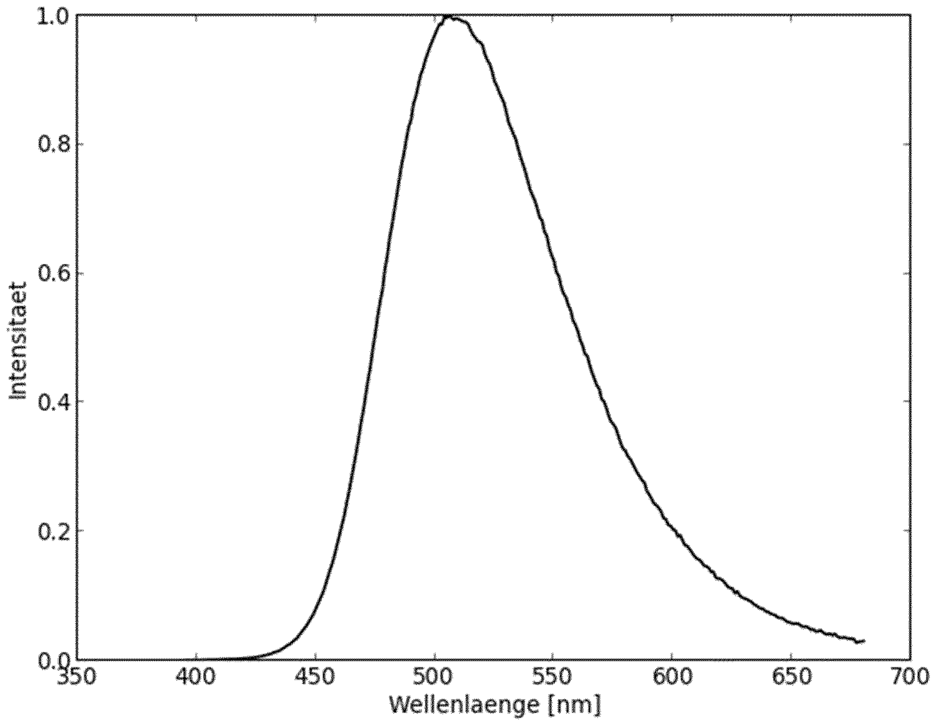

Figur 5

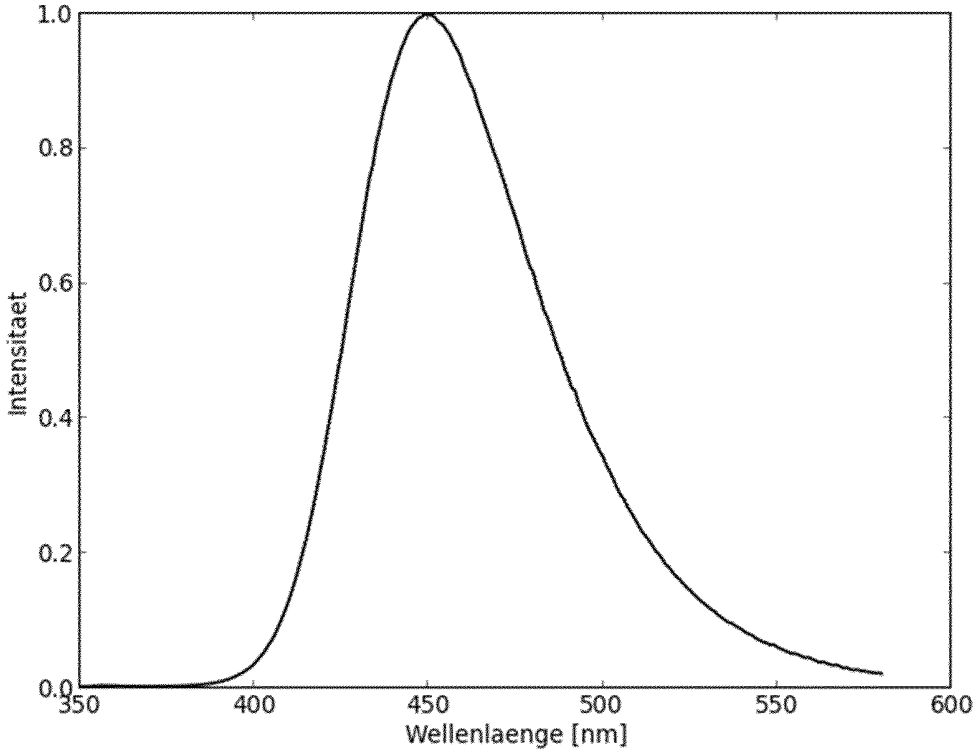


Figur 6

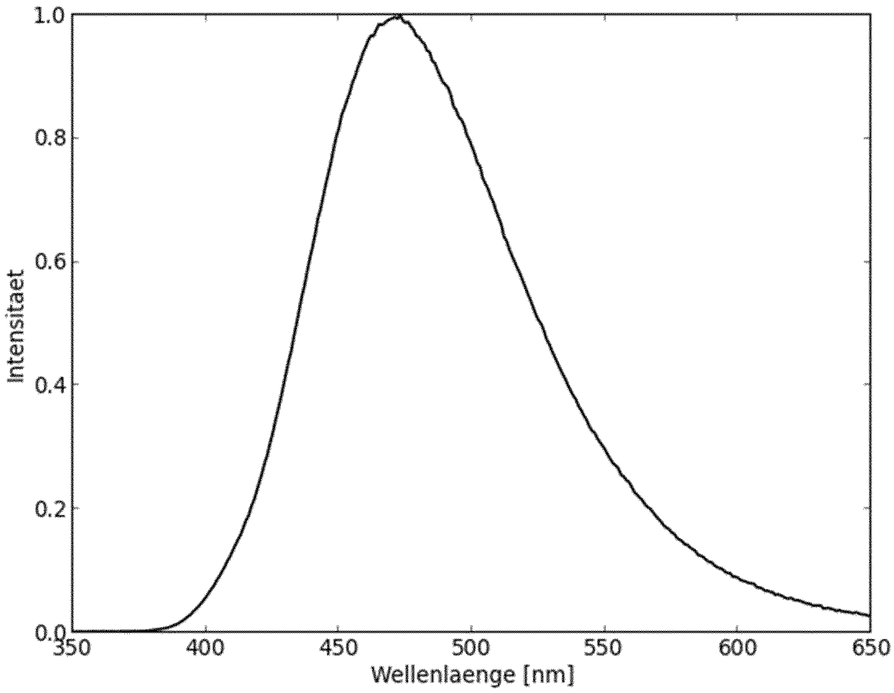

Figur 7

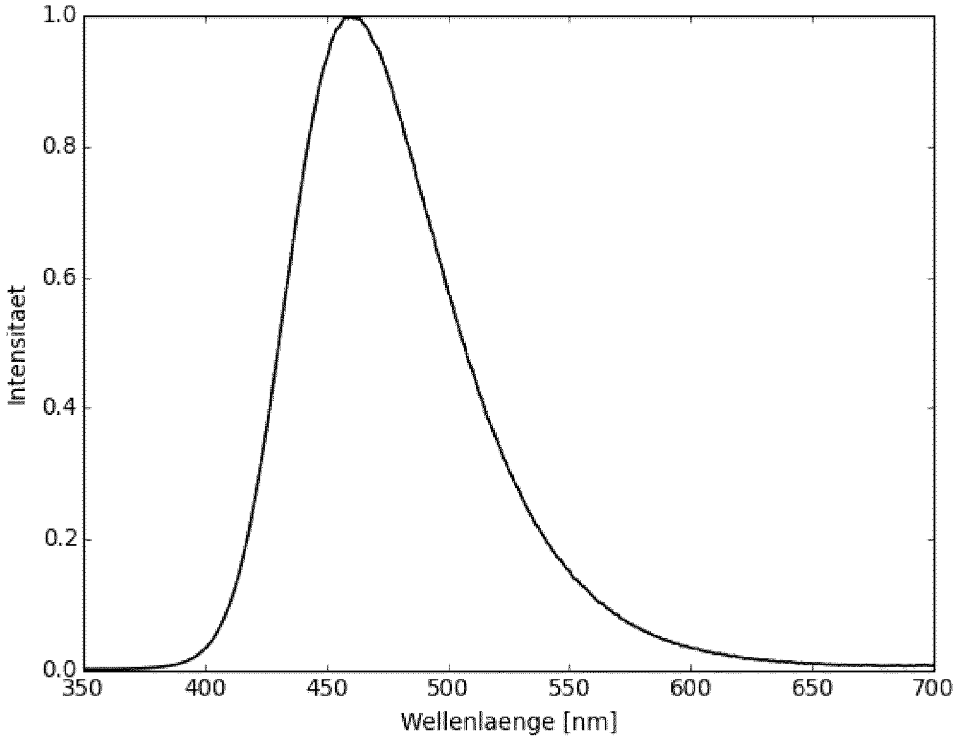


Figur 8

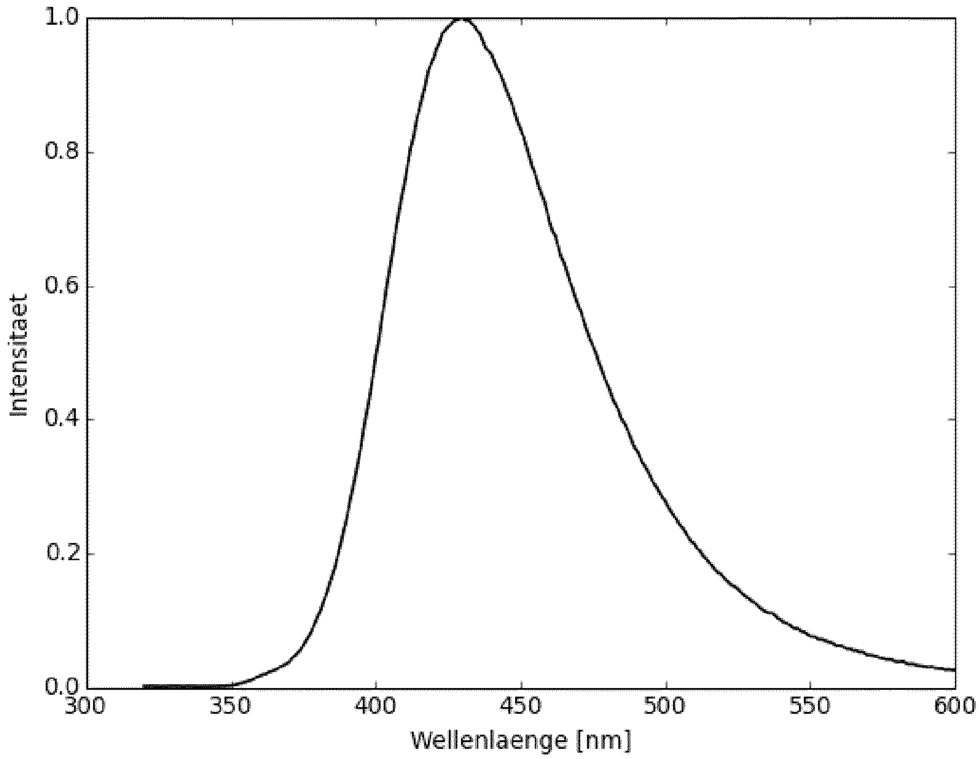

Figur 9

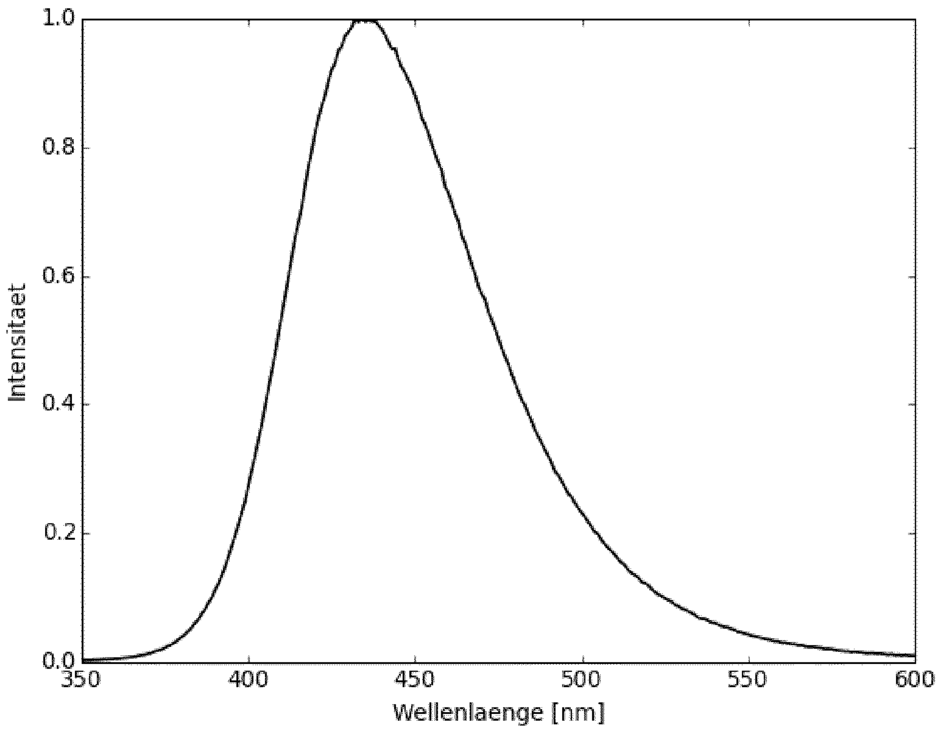


Figur 10

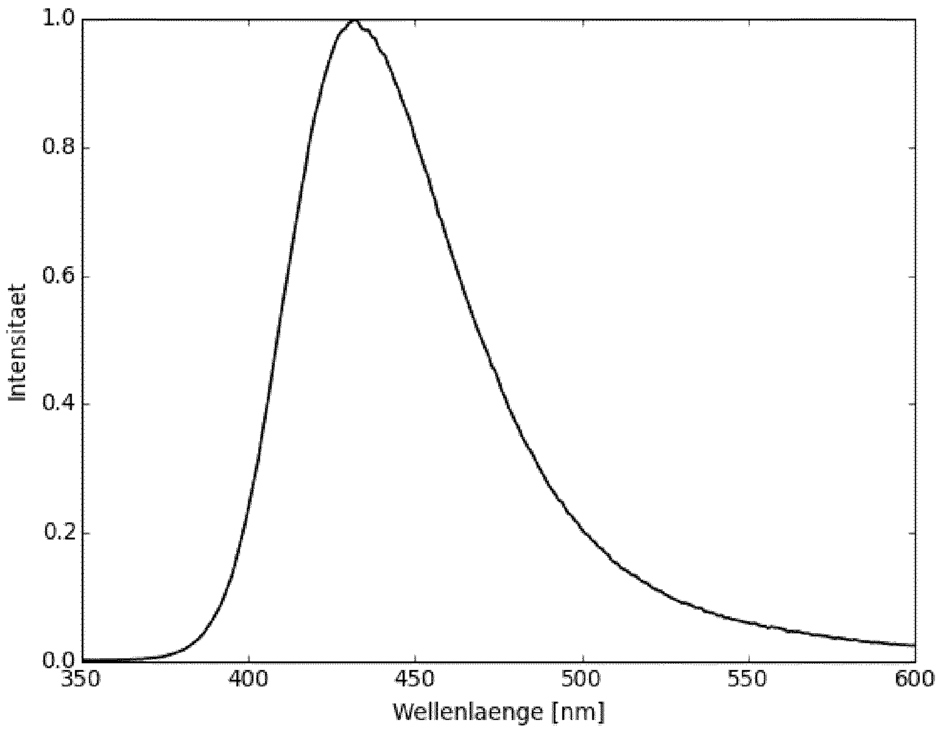

Figur 11

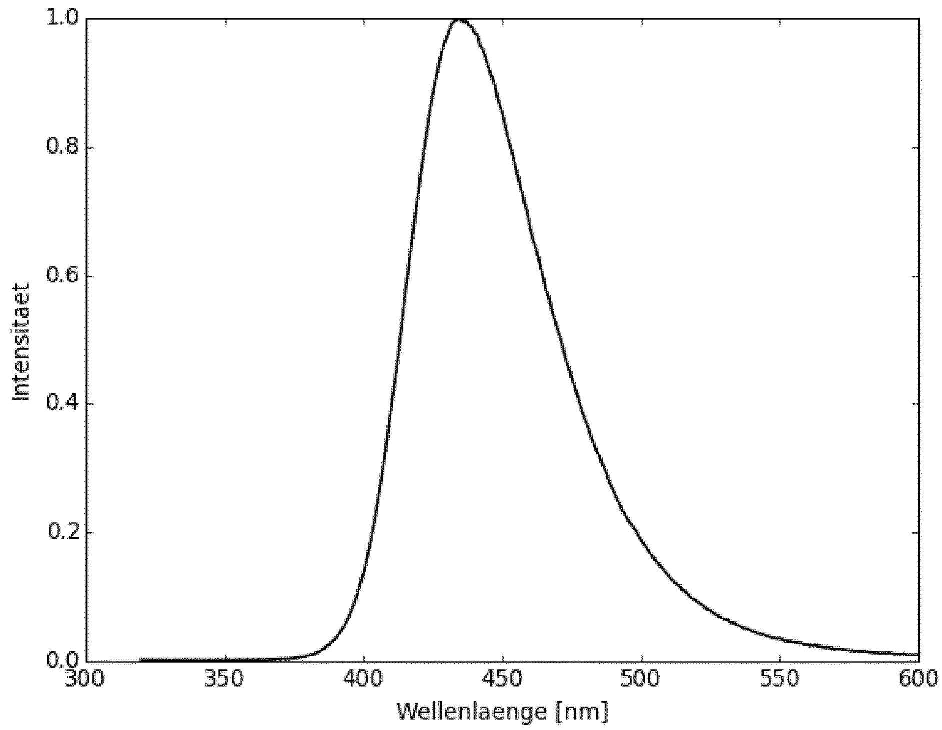


Figur 12

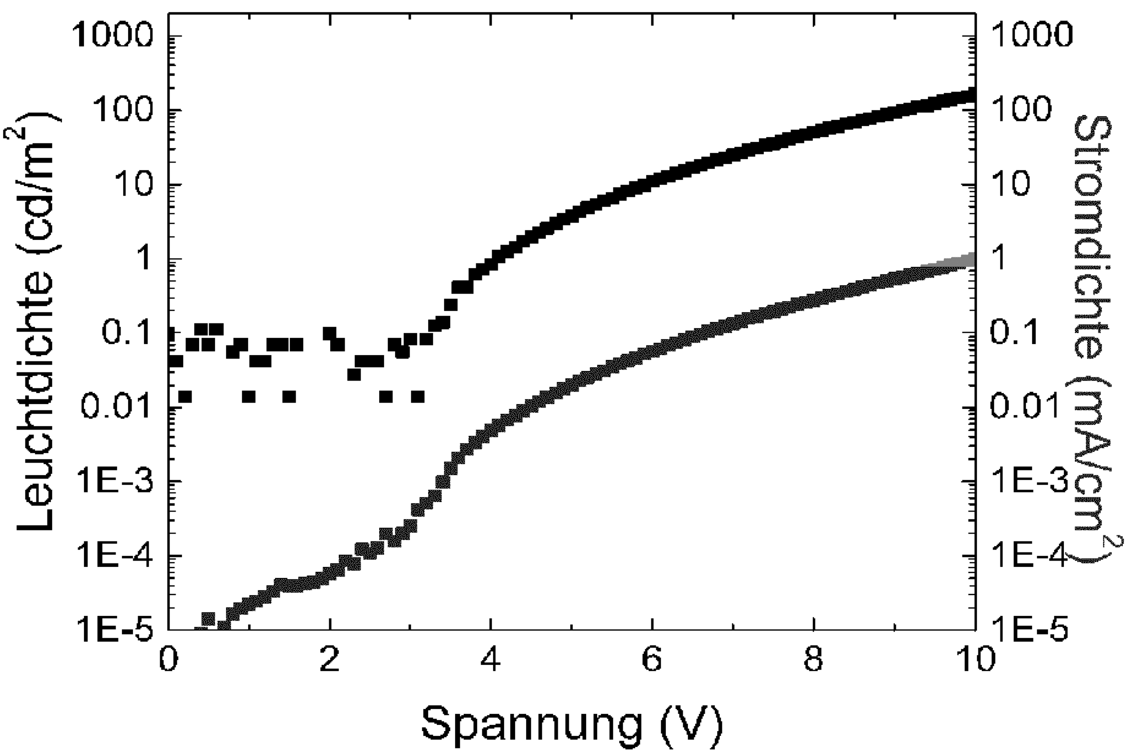

Figur 13

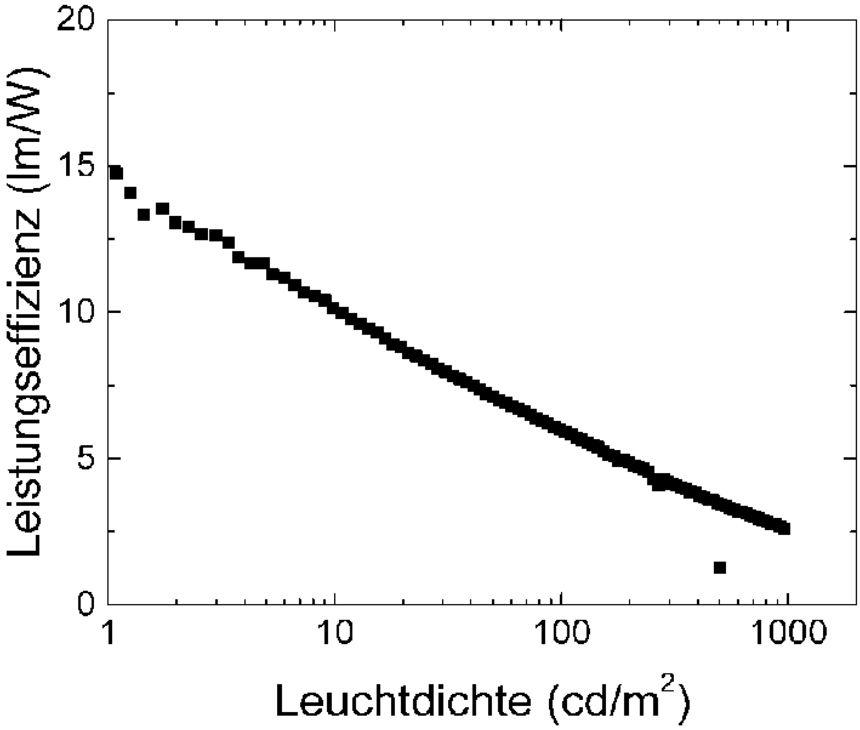


Figur 14

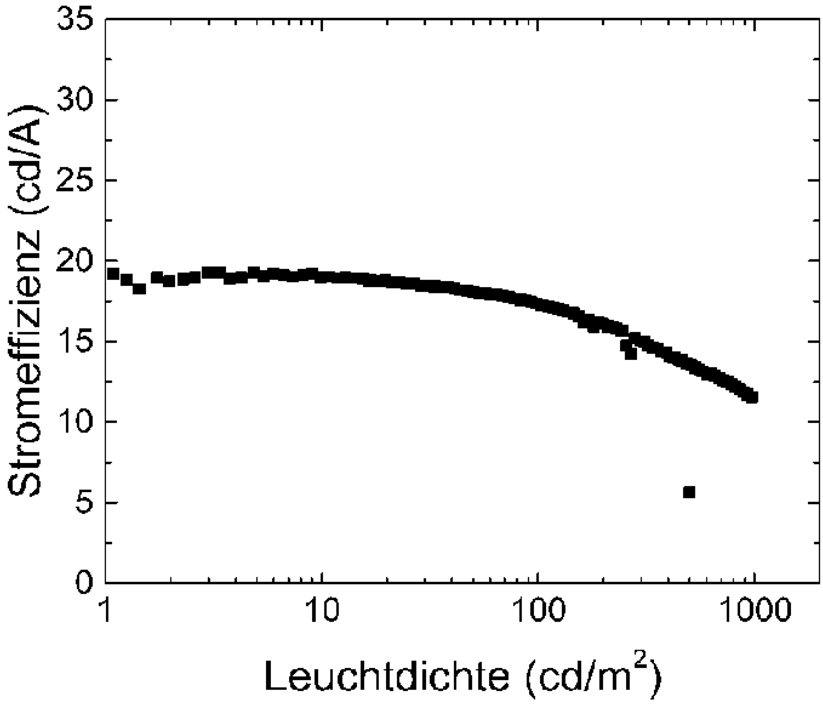

Figur 15

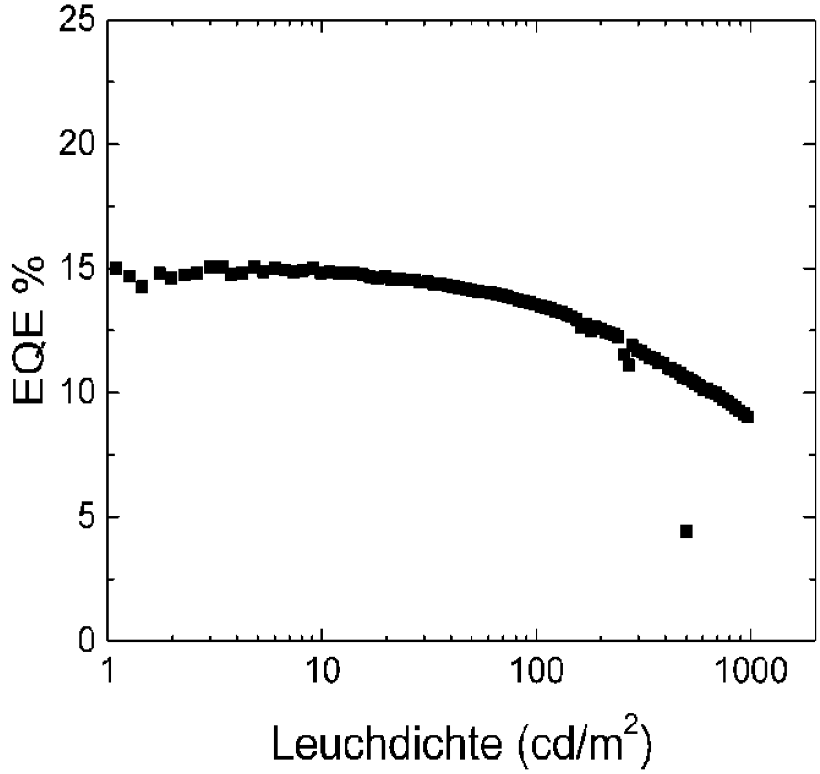


Figur 16

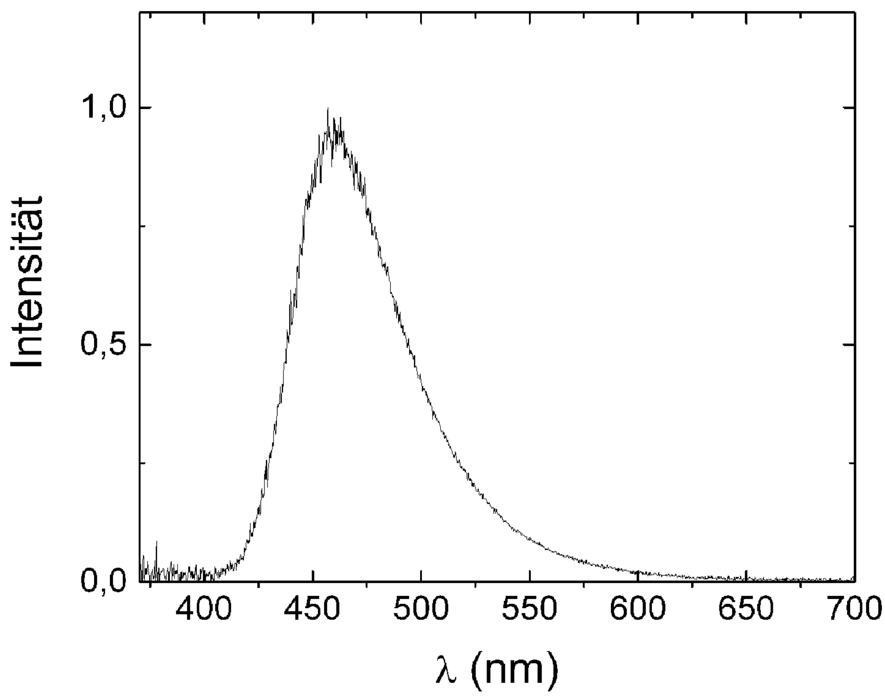

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010149748 A1, H. Yersin, U. Monkowius, T. Fischer, T. Hofbeck
- WO 2013161437 A1
- US 20140131686 A1


### In der Beschreibung aufgeführte Nicht-Patentliteratur


- **Q. ZHANG et al.** *J. Am. Chem. Soc.,* 2012, vol. 134, 14706
- **SHAOLONG GONG et al.** *J. Mater. Chem.,* 2012, vol. 22, 2894-2899
- **N. R. MADADI et al.** *Bioorg. Med. Chem. Lett.,* 2014, vol. 24, 601-603
- **M.E. THOMPSON et al.** *Chem. Mater.,* 2004, vol. 16, 4743
- **BECKE, A. D.** *Phys. Rev. A,* 1988, vol. 38, 3098-3100
- **PERDEW, J. P.** *Phys. Rev. B,* 1986, vol. 33, 8822-8827
- **SIERKA, M. ; HOGEKAMP, A. ; AHLRICHS, R.** *J. Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11